(19) **Europäisches Patentamt / European Patent Office / Office européen des brevets**

(11) **EP 3 766 541 B1**

(12) # EUROPEAN PATENT SPECIFICATION

(45) Date of publication and mention
of the grant of the patent:
**29.11.2023 Bulletin 2023/48**

(21) Application number: **19767606.7**

(22) Date of filing: **07.03.2019**

(51) International Patent Classification (IPC):
**A61N 5/10** *(2006.01)*

(52) Cooperative Patent Classification (CPC):
**A61N 5/1049; A61B 6/12; A61B 6/5264;
A61N 5/1067; G06F 30/27; G06T 7/0012;
G06T 7/246; G06T 7/292; G06V 10/225;
G06V 10/751; G06V 10/774; G06V 10/776;**
A61B 6/032; A61B 6/5223; A61B 6/587; (Cont.)

(86) International application number:
**PCT/JP2019/009134**

(87) International publication number:
**WO 2019/176734 (19.09.2019 Gazette 2019/38)**

(54) **MEDICAL IMAGE PROCESSING DEVICE, TREATMENT SYSTEM, AND MEDICAL IMAGE PROCESSING PROGRAM**

MEDIZINISCHE BILDVERARBEITUNGSVORRICHTUNG, BEHANDLUNGSSYSTEM UND
MEDIZINISCHES BILDVERARBEITUNGSPROGRAMM

DISPOSITIF DE TRAITEMENT D'IMAGE MÉDICALE, SYSTÈME DE TRAITEMENT ET
PROGRAMME DE TRAITEMENT D'IMAGE MÉDICALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.03.2018 JP 2018044793**

(43) Date of publication of application:
**20.01.2021 Bulletin 2021/03**

(73) Proprietor: **Toshiba Energy Systems & Solutions
Corporation
Saiwai-ku
Kawasaki-shi
Kanagawa 2120013 (JP)**

(72) Inventors:
• **HIRAI Ryusuke
Tokyo 105-0023 (JP)**
• **TAGUCHI Yasunori
Tokyo 105-0023 (JP)**
• **SAKATA Yukinobu
Tokyo 105-0023 (JP)**

• **OKAYA Keiko
Kawasaki-shi, Kanagawa 212-0013 (JP)**
• **MORI Shinichiro
Chiba-shi, Chiba 263-8555 (JP)**

(74) Representative: **Henkel & Partner mbB
Patentanwaltskanzlei, Rechtsanwaltskanzlei
Maximiliansplatz 21
80333 München (DE)**

(56) References cited:
WO-A1-2018/037608    JP-A- 2016 116 659
JP-A- 2016 131 737    JP-A- 2018 029 852
US-A1- 2012 093 397    US-A1- 2012 238 866

EP 3 766 541 B1

(52) Cooperative Patent Classification (CPC): (Cont.)
A61B 6/589; A61B 2034/2065; A61B 2090/376;
A61B 2090/3966; A61N 2005/1056;
G06T 2207/10064; G06T 2207/20081;
G06T 2207/30096; G06T 2207/30204;
G06V 2201/03

**Description**

[Technical Field]

Embodiments of the present invention relate to a medical image processing device, a treatment system, and a medical image processing program.

[Background Art]

Radiation treatment is a treatment method of irradiating a lesion within a patient's body with radiation to destroy the lesion. At this time, the radiation is required to be accurately radiated to the position of the lesion. This is because, when normal tissue within the patient's body is irradiated with the radiation, the normal tissue may be affected. Thus, when the radiation treatment is performed, computed tomography (CT) is first performed in advance in a treatment planning stage and the position of the lesion within the patient's body is three-dimensionally ascertained. The radiation irradiation direction and the radiation irradiation intensity are planned to reduce irradiation for the normal tissue on the basis of the ascertained position of the lesion. Thereafter, the position of the patient in a treatment stage is aligned with the position of the patient planned in the treatment planning stage and a lesion is irradiated with radiation in accordance with the irradiation direction or the irradiation intensity planned in the treatment planning stage.

In the position alignment of the patient in the treatment stage, image collation between a fluoroscopic image of the inside of the patient's body captured in a state in which the patient is laid on the bed immediately before the start of treatment and a digitally reconstructed radiograph (DRR) image in which the fluoroscopic image is virtually reconstructed from a three-dimensional CT image captured at the time of the treatment planning is performed and position deviation of the patient between images is obtained. The bed is moved on the basis of the obtained position deviation of the patient. Thereby, the position of a lesion, bone, or the like within the patient's body is aligned with that planned in the treatment planning.

The position deviation of the patient is obtained by seeking a position in the CT image so that the DRR image most similar to the fluoroscopic image is reconstructed. Many methods of automatically seeking the position of a patient using a computer have been proposed. However, a user (a doctor or the like) finally confirms an automated seeking result by comparing the fluoroscopic image with the DRR image. As soon as the confirmation by the user (the doctor or the like) is obtained, the irradiation with radiation is performed.

However, the lesion within the patient's body may be an organ, which moves due to the motion of the patient's respiration or heartbeat, such as the lung or liver. In this case, the location of the lesion must be identified during the irradiation with radiation. As a method of identifying the location of the lesion, there is a method of capturing a fluoroscopic moving image of a patient during irradiation with radiation and tracking a lesion within the patient's body on the basis of a fluoroscopic image. Also, there is a method of indirectly identifying the position of a lesion by tracking a marker placed within a patient's body according to percutaneous surgery in a case in which the lesion in the patient's body is not clearly visible in a fluoroscopic image or the like. Radiation irradiation methods are tracking irradiation in which the position of a lesion is tracked and the lesion is irradiated with radiation, ambush irradiation in which the lesion is irradiated with radiation when the lesion reaches the position planned in the treatment planning, and the like. These radiation irradiation methods are referred to as respiratory synchronous irradiation methods.

The marker placed within the patient's body is made of a metallic substance and is highly visible because it appears dark in the fluoroscopic image. This is because X-rays and the like used when a fluoroscopic image is captured tend to be absorbed by metals. Thus, in tracking of the marker, an image obtained by photographing the marker is provided as a template in advance and the position of the marker within the fluoroscopic image captured in the treatment stage is detected according to template matching.

Incidentally, there are various shapes such as a spherical shape, a rod shape, and a wedge shape for the shape of the marker placed within the patient's body. When the marker has a spherical shape, an image of the marker shown within the fluoroscopic image is circular regardless of the posture of the marker in the three-dimensional space. Thus, the position of the spherical marker can be easily detected according to template matching. However, the spherical marker may move in the patient's daily life. That is, when the marker is spherical, there may be deviation between the position planned in the treatment planning stage and the position at the time of the treatment stage. If the position of the marker deviates, the position of the lesion will not be accurately irradiated with the radiation radiated during the treatment stage. For this reason, in recent years, the number of opportunities in which a non-spherical marker such as a rod-shaped marker or a wedge-shaped marker that is easily retained within the patient's body is adopted has increased. However, when the marker is rod- or wedge-shaped, the image of the marker shown within the fluoroscopic image changes in various shapes depending on a posture of the marker within a three-dimensional space. Thus, the detection of the position of a rod- or wedge-shaped marker according to template matching is more difficult than the detection of the position of a spherical marker.

Thus, for example, a method disclosed in Patent Document 1 is an example of a method of tracking a marker shown within a fluoroscopic image. In the method disclosed in Patent Document 1, a plurality of marker images photographed at various angles are provided as templates and the position of a marker within the fluoroscopic image captured in the treatment stage is detected according to template matching with each template. However, the method disclosed in Patent Document 1 requires calculation of degrees of similarity between a large number of templates and the marker within the fluoroscopic image. Thus, the method disclosed in Patent Document 1 has a problem in that real-time performance for tracking the marker becomes low. Also, in the method disclosed in Patent Document 1, because a template that is significantly different from the actual marker image may also be used for template matching, there may also be a template having a high degree of similarity to the image other than the marker included in the fluoroscopic image. In this case, in the method disclosed in Patent Document 1, a possibility that an image other than the marker included in the fluoroscopic image will be detected and tracked as the marker may increase.

Patent Document 2 discloses a medical image processing apparatus including a first image acquirer that acquires a plurality of first images of an object to be treated, each of the plurality of first images being captured by a radiographic imaging apparatus at a respective time of a plurality of times; a second image acquirer that acquires a second image of the object to be treated, the second image being captured by a radiographic imaging apparatus at a time different from the plurality of times when the plurality of first images are captured; a characteristic processor that determines a plurality of positions of a first characteristic of the object to be treated from the plurality of first images, the characteristic processor that determines the position of a second characteristic corresponding to the first characteristic in the object to be treated from the second image; a calculator that establishes a first closed space including the plurality of positions of the first characteristic; and a determiner that determines whether or not the position of the second characteristic is within the first closed space, and the determiner that outputs a determination signal, based on a result of the determination.

Patent Document 3 discloses a method and system for adaptive discriminant learning and measurement fusion for image based catheter tracking. An adaptive discriminant model is trained online based on a tracked object, such as a pigtail catheter tip, in at least one previous frame of a fluoroscopic image sequence. The object is tracked in the current frame of the fluoroscopic image sequence based at least on the adaptive discriminant model trained online. The object may be tracked in the current frame based on a fusion of three types of measurement models including the adaptive discriminant model trained online, an object detection model trained offline, and an online appearance model.

Patent Document 4 discloses methods and systems for training a learning based classifier and object detection in medical images. In order to train a learning based classifier, positive training samples and negative training samples are generated based on annotated training images. Features for the positive training samples and the negative training samples are extracted. The features include an extended Haar feature set including tip features and corner features. A discriminative classifier is trained based on the extracted features.

[Citation List]

[Patent Literature]

[Patent Document 1]
Japanese Unexamined Patent Application, First Publication No. 2016-131737
[Patent Document 2]
WO 2018/037608 A1
[Patent Document 3]
US 2012/238866 A1
[Patent Document 4]
US 2012/093397 A1

[Summary of Invention]

[Technical Problem]

An objective of an aspect of the present invention is to provide a medical image processing device, a treatment system, and a medical image processing program capable of automatically tracking a marker placed within a patient's body in a fluoroscopic image of the patient who is being irradiated with radiation in radiation treatment.

[Solution to Problem]

Accordingly, there is provided a medical image processing device as set out in independent claim 1, a treatment system as set out in claim 14, and a medical image processing program as set out in independent claim 16. Advantageous developments are defined in the dependent claims.

[Advantageous Effects of Invention]

According to the above-described aspect, it is possible to provide a medical image processing device, a treatment system, and a medical image processing program capable of automatically tracking a marker placed within a patient's body in a fluoroscopic image of the patient who is being irradiated with radiation in radiation treatment.

[Brief Description of Drawings]

Fig. 1 is a block diagram showing a schematic configuration of a treatment system including a medical image processing device of a first embodiment.
Fig. 2 is a block diagram showing a schematic configuration of the medical image processing device of the first embodiment.
Fig. 3 is a flowchart showing a flow of an operation of a learning device constituting the medical image processing device of the first embodiment.
Fig. 4 is a flowchart showing a flow of an operation of a moving object tracking device constituting the medical image processing device of the first embodiment.
Fig. 5 is a diagram for describing a relationship between a radiation irradiation path and a marker in the treatment system including the medical image processing device of the first embodiment.
Fig. 6 is a block diagram showing a schematic configuration of a medical image processing device of a second embodiment.
Fig. 7A is a flowchart showing a flow of an operation of a learning device constituting the medical image processing device of the second embodiment.
Fig. 7B is a flowchart showing a flow of an operation of the learning device constituting the medical image processing device of the second embodiment.
Fig. 8 is a diagram showing an example of an image of a marker extracted by the learning device constituting the medical image processing device of the second embodiment.
Fig. 9 is a block diagram showing a schematic configuration of a medical image processing device of a third embodiment.
Fig. 10 is a diagram showing information displayed by the medical image processing device of the third embodiment.
Fig. 11 is a block diagram showing a schematic configuration of a medical image processing device of a fourth embodiment.
Fig. 12A is a flowchart showing a flow of an operation of a moving object tracking device constituting the medical image processing device of the fourth embodiment.
Fig. 12B is a flowchart showing a flow of an operation of the moving object tracking device constituting the medical image processing device of the fourth embodiment.
Fig. 13 is a diagram showing a template used for tracking a marker in the moving object tracking device constituting the medical image processing device of the fourth embodiment.
Fig. 14 is a block diagram showing a schematic configuration of a medical image processing device of a fifth embodiment.

[Description of Embodiments]

Hereinafter, a medical image processing device, a treatment system, and a medical image processing program of embodiments will be described with reference to the drawings.

(First embodiment)

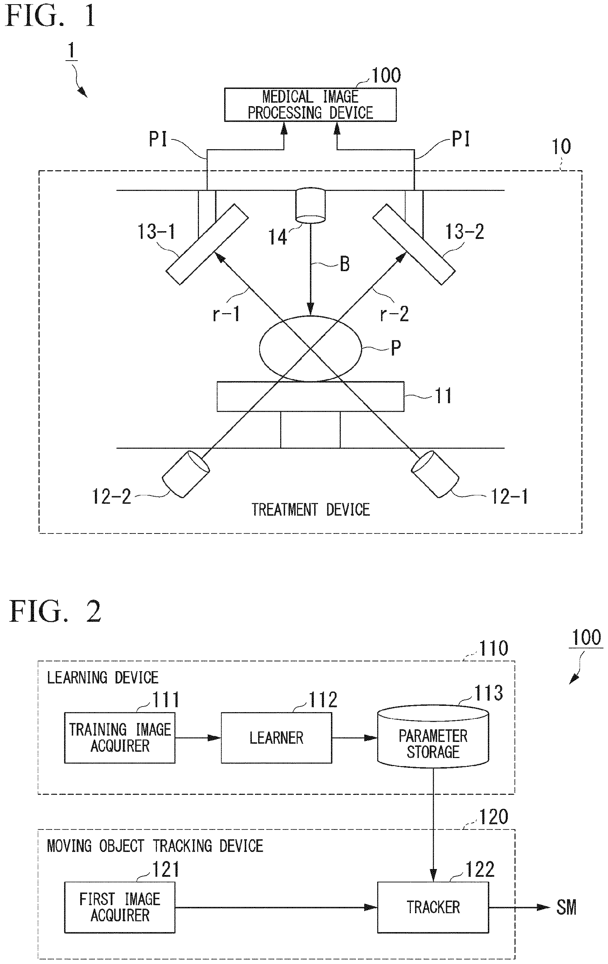

Fig. 1 is a block diagram showing a schematic configuration of a treatment system including a medical image processing device of a first embodiment. A treatment system 1 shown in Fig. 1 includes a medical image processing device 100 and a treatment device 10.

First, the treatment device 10 constituting the treatment system 1 will be described. The treatment device 10 includes a treatment table 11, two radiation sources 12 (a radiation source 12-1 and a radiation source 12-2), two radiation detectors 13 (a radiation detector 13-1 and a radiation detector 13-2), and a treatment beam irradiation gate 14.

Also, a hyphen "-" indicated after a reference numeral shown in Fig. 1 and a number subsequent to the hyphen are used for identifying an associated relationship. For example, in the associated relationship between the radiation source 12 and the radiation detector 13 in the treatment device 10, a state in which the radiation source 12-1 and the radiation detector 13-1 correspond to each other to form one set is shown and a state in which the radiation source 12-2 and the radiation detector 13-2 correspond to each other to form another set is shown. That is, in the following description, the hyphen "-" indicated after each reference numeral and a number subsequent to the hyphen represent that components of the same set correspond to each other. In the following description, when a plurality of identical components are represented without being distinguished, they are represented without the hyphen "-" and the number subsequent to the hyphen.

The treatment table 11 is a bed on which a subject (a patient) P which will undergo radiation treatment is fixed.

The radiation source 12-1 radiates radiation r-1 for seeing through the body of the patient P at a predetermined angle. The radiation source 12-2 radiates radiation r-2 for seeing through the body of the patient P at a predetermined angle different from that of the radiation source 12-1. The radiation r-1 and the radiation r-2 are, for example, X-rays. In Fig. 1, a case in which X-ray photography is performed in two directions on the patient P fixed on the treatment table 11 is shown. Also, the illustration of a controller that controls the irradiation with the radiation r by the radiation source 12 is omitted from Fig. 1.

The radiation detector 13-1 detects the radiation r-1 which has been radiated from the radiation source 12-1 and has arrived at the radiation detector 13-1 after passing through the inside of the body of the patient P and generates a fluoroscopic image PI of the inside of the body of the patient P according to a magnitude of energy of the detected radiation r-1. The radiation detector 13-2 detects the radiation r-2 which has been radiated from the radiation source 12-2 and has arrived at the radiation detector 13-2 after passing through the inside of the body of the patient P and generates a fluoroscopic image PI of the inside of the body of the patient P according to a magnitude of energy of the detected radiation r-2. The radiation detectors 13 are detectors arranged in a two-dimensional array shape and generate a digital image in which a magnitude of energy of the radiation r arriving at each detector is represented by a digital value as a fluoroscopic image PI. The radiation detector 13 is, for example, a flat panel detector (FPD), an image intensifier, or a color image intensifier. The radiation detector 13 outputs the generated fluoroscopic image PI to the medical image processing device 100. Also, the illustration of a controller that controls the generation of the fluoroscopic image PI by the radiation detector 13 is omitted from Fig. 1.

In the treatment device 10, a set of a radiation source 12 and a radiation detector 13 constitutes an imaging device in the treatment system 1.

Also, in Fig. 1, a configuration of the treatment device 10 including two sets of radiation sources 12 and radiation detectors 13, i.e., two imaging devices, is shown. However, the number of imaging devices provided in the treatment device 10 is not limited to a configuration including two imaging devices as shown in Fig. 1, i.e., a configuration including two sets of the radiation sources 12 and the radiation detectors 13. For example, the treatment device 10 may be configured to include three or more imaging devices (three or more sets of radiation sources 12 and radiation detectors 13). Also, the treatment device 10 may be configured to include one imaging device (a set of a radiation source 12 and a radiation detector 13).

The treatment beam irradiation gate 14 radiates radiation as a treatment beam B for destroying a lesion, which is a target portion to be treated within the body of the patient P. The treatment beam B is, for example, X-rays, γ-rays, an electron beam, a proton beam, a neutron beam, a heavy particle beam, or the like. Also, the illustration of a controller that controls the irradiation with the treatment beam B by the treatment beam irradiation gate 14 is omitted from Fig. 1.

Also, in Fig. 1, the configuration of the treatment device 10 including one treatment beam irradiation gate 14 is shown. However, the treatment device 10 is not limited to the configuration including only one treatment beam irradiation gate 14 and may include a plurality of treatment beam irradiation gates. For example, although the configuration of the treatment device 10 including the treatment beam irradiation gate 14 for irradiating the patient P with the treatment beam B in a vertical direction is shown in Fig. 1, the treatment system 1 may further include a treatment beam irradiation gate for irradiating the patient P with the treatment beam in a horizontal direction.

The medical image processing device 100 is used as a respiratory synchronous irradiation device that performs radiation treatment in a respiratory synchronous irradiation method of radiating a treatment beam B to an organ, which moves due to the motion of respiration or heartbeat of the patient P, such as the lung or the liver. The medical image processing device 100 tracks a marker placed within the body of the patient P to be treated in the radiation treatment on the basis of the fluoroscopic images PI output from the radiation detector 13-1 and the radiation detector 13-2. Thereby, the medical image processing device 100 can track the lesion within the body of the patient P to be treated in the radiation treatment, for example, from a relationship between relative positions of the marker and the lesion and the like. The tracking of the lesion in the medical image processing device 100 is performed by tracking the position of the marker photographed in the fluoroscopic image PI of the patient P at present on the basis of features of an image of the marker (hereinafter referred to as a "marker image") learned before the radiation treatment is performed as in the treatment planning stage or the like. The medical image processing device 100 automatically detects a timing at which the lesion will be irradiated with the treatment beam B in the radiation treatment on the basis of a result of tracking the marker placed within the body of the patient P.

Also, the medical image processing device 100 and the radiation detector 13 provided in the treatment device 10 may be connected by a local region network (LAN) or a wide region network (WAN).

Subsequently, the configuration of the medical image processing device 100 constituting the treatment system 1 will be described. Fig. 2 is a block diagram showing a schematic configuration of the medical image processing device 100 of the first embodiment. The medical image processing device 100 shown in Fig. 2 includes a learning device 110 and a moving object tracking device 120. The learning device 110 also includes a training image acquirer 111, a learner 112, and a parameter storage 113. Also, the moving object tracking device 120 includes a first image acquirer 121 and a tracker 122.

The learning device 110 acquires a training image in the medical image processing device 100 before the radiation treatment is performed and learns a first feature for detecting a marker image from the acquired training image. Also, the learning device 110 calculates a feature extraction parameter representing the feature of the marker on the basis of the learned first feature and causes the feature extraction parameter to be stored in the parameter storage 113.

The training image acquirer 111 acquires a training image in the medical image processing device 100. The training image acquirer 111 outputs the acquired training image to the learner 112. Here, the training image is a plurality of images created in the treatment planning stage when the radiation treatment is performed or a stage before the treatment planning stage. The training image is created by combining an image of a marker, which is an object arranged in a virtual three-dimensional space, with a clinical image of a portion where the radiation treatment is performed. More specifically, the training image is created by combining simulation images obtained by performing simulation in various directions when a target marker to be tracked is placed within the patient's body with a clinical image for each portion on which the radiation treatment is performed. The clinical image for generating the training image may be a digitally reconstructed radiograph (DRR) image obtained by virtually reconstructing the fluoroscopic image PI from a three-dimensional computed tomography (CT) image captured at the time of the treatment planning. Thereby, the training image becomes an image obtained by performing simulation in all available directions of the marker image of the marker placed within the patient's body. The simulation image and the clinical image are combined by the existing image compositing technology such as alpha blending.

The learner 112 acquires a plurality of training images from the training image acquirer 111 and learns the first feature common to marker images included within the training images as object images from the plurality of acquired training images. Here, the learner 112 learns a plurality of first features obtained when the marker is observed in all directions from the plurality of training images. Also, the learning of the first feature of the marker in the learner 112 is performed using, for example, the existing machine learning technology. The learner 112 calculates a feature extraction parameter representing the feature in the direction of the marker on the basis of the first feature obtained through learning. The learner 112 outputs the calculated feature extraction parameter to the parameter storage 113 so that the parameter storage 113 is allowed to store the feature extraction parameter.

The parameter storage 113 is a storage device that stores the feature extraction parameters output from the learner 112. The parameter storage 113 outputs the stored feature extraction parameter to the tracker 122 in response to a request from the tracker 122 provided in the moving object tracking device 120.

The moving object tracking device 120 acquires a first image, which is a fluoroscopic image PI of the patient P in which the marker to be tracked by the medical image processing device 100 is photographed, when the radiation treatment is performed, and tracks the marker photographed in the first image on the basis of a first feature of the marker learned by the learning device 110. Also, the moving object tracking device 120 outputs a marker position signal SM representing the position of the marker which is being tracked.

The first image acquirer 121 acquires the first image which is the fluoroscopic image PI of the patient P captured during treatment. The first image acquirer 121 outputs the acquired first image to the tracker 122. Here, the first image is the fluoroscopic image PI of the inside of the body of the patient P photographed at a predetermined time interval in a state in which the patient P is laid on the treatment table 11 during the treatment. That is, the first image is a fluoroscopic image PI generated by the radiation detector 13 in accordance with radiation r radiated by the radiation source 12 during the treatment (in which the treatment beam irradiation gate 14 may radiate the treatment beam B or may not radiate the treatment beam B). Also, the first image acquirer 121 may include an interface for connecting to the radiation detector 13 provided in the treatment device 10.

The tracker 122 tracks the marker photographed as the object in the first image output from the first image acquirer 121. The tracker 122 acquires the feature extraction parameter stored in the parameter storage 113 provided in the learning device 110 when the marker is tracked. The tracker 122 calculates (extracts) a feature similar to the first feature of the marker image included as the object image in the first image output from the first image acquirer 121 on the basis of the acquired feature extraction parameter. Also, in the following description, for ease of description, a feature similar to the first feature calculated (extracted) by the tracker 122 is also referred to as a "first feature." The tracker 122 tracks the marker photographed in the first image on the basis of the calculated first feature. The tracker 122 outputs a marker position signal SM representing the position of the marker which is being tracked.

According to this configuration, the medical image processing device 100 tracks the marker within the body of the patient P at present on the basis of the learned feature of the marker and outputs the marker position signal SM representing the position of the tracked marker. Thereby, the medical image processing device 100 can automatically detect a timing at which the lesion within the body of the patient P will be irradiated with the treatment beam B. Thus, the treatment system 1 including the medical image processing device 100 can irradiate the lesion within the body of the patient P with the treatment beam B at an appropriate timing. Also, the treatment system 1 performs control so that the treatment beam B is radiated when the position of the marker which is being tracked, i.e., the position of the lesion within the body of the patient P capable of being identified from the position of the marker, is within a predetermined range. In other words, the treatment system 1 performs control so that the irradiation with the treatment beam B is stopped when the position of the lesion within the body of the patient P is not within the predetermined range.

Also, some of the functional units provided in the medical image processing device 100 described above may be software function units that function by a processor, for example, such as a central processing unit (CPU) or a graphics processing unit (GPU), executing a program stored in a storage device. Here, the storage device may be implemented by a read only memory (ROM), a random-access memory (RAM), a hard disk drive (HDD), a flash memory, or the like. The program executed by the processor such as a CPU or a GPU may be pre-stored in the storage device of the medical image processing device 100 or may be downloaded from another computer device via a network. Also, the program stored in the portable storage device may be installed in the medical image processing device 100. Also, some or all of the functional units provided in the above-described medical image processing device 100 may be hardware function units based on a field programmable gate array (FPGA), a large-scale integration (LSI) circuit, an application specific integrated circuit (ASIC), and the like.

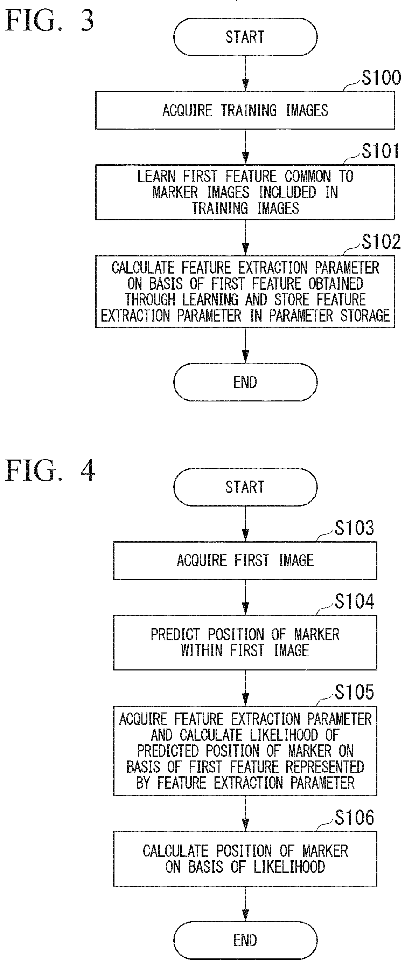

Here, the operation of the medical image processing device 100 constituting the treatment system 1 will be schematically described. First, the operation of the learning device 110 constituting the medical image processing device 100 will be schematically described. Fig. 3 is an example of a flowchart showing a flow of the operation of the learning device 110 constituting the medical image processing device 100 of the first embodiment. Also, the learning device 110 in the medical image processing device 100 causes the parameter storage 113 to store a feature extraction parameter before the radiation treatment is performed.

When the learning device 110 starts an operation, the training image acquirer 111 first acquires training images (step S100). Subsequently, the learner 112 learns a first feature common to marker images included in the training images output from the training image acquirer 111 (step S101). Subsequently, the learner 112 calculates a feature extraction parameter on the basis of the first feature obtained through learning, outputs the calculated feature extraction parameter to the parameter storage 113, and causes the parameter storage 113 to store the calculated feature extraction parameter (step S102).

Subsequently, the operation of the moving object tracking device 120 constituting the medical image processing device 100 will be schematically described. Fig. 4 is an example of a flowchart showing a flow of the operation of the moving object tracking device 120 constituting the medical image processing device 100 of the first embodiment. Also, in the medical image processing device 100, the moving object tracking device 120 tracks a marker actually placed within the patient's body on the basis of the feature extraction parameter when radiation treatment is performed.

When the moving object tracking device 120 starts an operation, the first image acquirer 121 first acquires a first image (step S103). Subsequently, the tracker 122 predicts the position of a marker within the first image output from the first image acquirer 121 (step S104). Subsequently, the tracker 122 acquires a feature extraction parameter stored in the parameter storage 113 and calculates the likelihood of the predicted marker position on the basis of a first feature represented by the acquired feature extraction parameter (step S105). Subsequently, the tracker 122 calculates the position of the marker included in the first image on the basis of the calculated likelihood (step S106).

Next, details of the operation of the medical image processing device 100 constituting the treatment system 1 will be described. First, a training image acquisition method for use in the learning device 110 constituting the medical image processing device 100 will be described.

Also, the shape of the target marker to be tracked by the tracker 122 has various shapes such as a spherical shape, a rod shape, a wedge shape, and a clip shape. More specifically, the marker is, for example, a spherical metallic marker having a diameter of 2 mm, or a rod-shaped metallic marker having a diameter of 0.5 mm and a length of 5 mm, 7 mm, or 10 mm. If the marker placed within the body of the patient P is a spherical marker, the marker image photographed in the fluoroscopic image PI of the patient P captured when radiation treatment is performed is circular regardless of a posture of the marker within a three-dimensional space. On the other hand, if the marker placed within the body of the patient P is a rod-shaped marker, the marker image photographed in the fluoroscopic image PI of the patient P captured when radiation treatment is performed is changed in various shapes according to the posture of the marker within the three-dimensional space. In the following description, a case in which the training image acquirer 111 acquires a training image of a marker other than a spherical marker placed within the patient's body and acquires a training image of a rod-shaped marker as an example will be described.

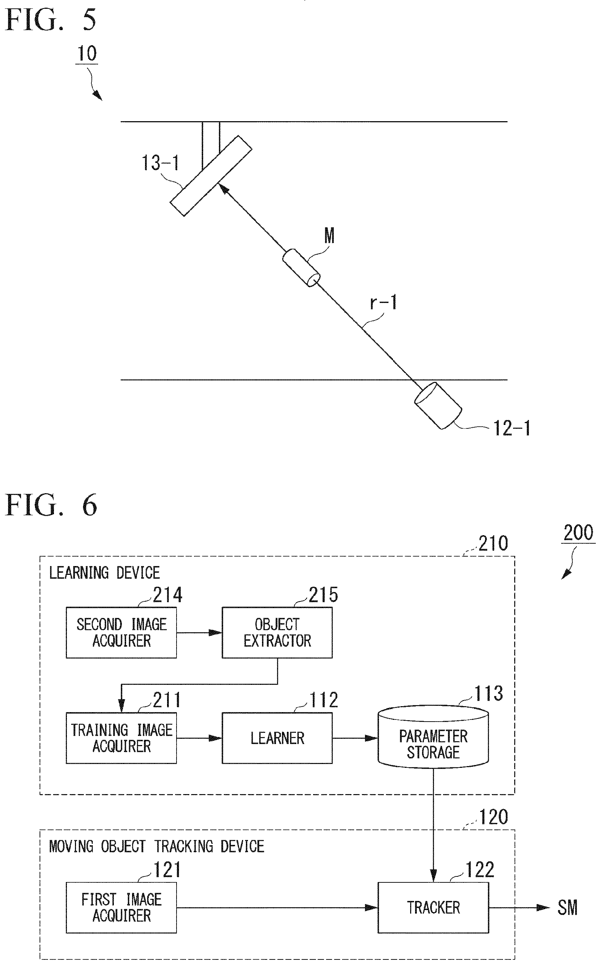

Fig. 5 is a diagram for describing an example of a relationship between a radiation irradiation path and a marker (a rod-shaped marker) in the treatment system 1 including the medical image processing device 100 of the first embodiment. Fig. 5 is a diagram showing an extracted positional relationship between the radiation source 12-1 and the radiation detector 13-1 in the treatment device 10 shown in Fig. 1. As shown in Fig. 5, in the three-dimensional space between the radiation source 12-1 and the radiation detector 13-1, the marker image of the rod-shaped marker M becomes circular when the rod-shaped marker M is placed within the body of the patient P so that the length direction of the rod-shaped marker M is parallel to a direction normal to a photography plane of the radiation detector 13-1 (a direction of the irradiation path of the radiation r-1 radiated by the radiation source 12-1). When the rod-shaped marker M is tilted from the state shown in Fig. 5, the marker image of the rod-shaped marker M gradually becomes longer with a feature in the length direction. Also, this feature that the shape of the marker image changes in various different types in accordance with the posture of the marker is similar to a feature in a wedge-shaped marker or a clip-shaped marker, i.e., a marker other than a spherical marker, as well as a feature in only the rod-shaped marker M.

In the learning device 110, a first feature serving as a feature common to markers is learned so that a marker image whose shape changes in various different types according to the posture of the marker is detected from the inside of the fluoroscopic image PI of the inside of the body of the patient P.

Also, in Fig. 5, an example of the rod-shaped marker M photographed by the imaging device including the set of the radiation source 12-1 and the radiation detector 13-1 is shown. However, as shown in Fig. 1, the treatment system 1 includes two imaging devices. Accordingly, the learning device 110 learns two first features for detecting marker images from fluoroscopic images PI obtained by simultaneously photographing the same rod-shaped marker M with the two imaging devices included in the treatment system 1 as one pair of first features. That is, the learning device 110 learns a first feature for detecting the marker image from a fluoroscopic image PI captured by the set of the radiation source 12-1 and the radiation detector 13-1 and a first feature for detecting the marker image from a fluoroscopic image PI captured by the set of the radiation source 12-2 and the radiation detector 13-2 as one set of first features. For example, when the rod-shaped marker M is placed within the body of the patient P as shown in Fig. 5 in the three-dimensional space, the marker image of the rod-shaped marker M in the fluoroscopic image PI captured by the set of the radiation source 12-1 and the radiation detector 13-1 becomes circular as described above. On the other hand, in this case, in the fluoroscopic image PI captured by the set of the radiation source 12-2 and the radiation detector 13-2, the marker image of the rod-shaped marker M becomes a rectangle having the longest length. Accordingly, the learning device 110 learns the first feature for detecting a set of a circular marker image captured by the set of the radiation source 12-1 and the radiation detector 13-1 and a rectangular marker image captured by the set of the radiation source 12-2 and the radiation detector 13-2 from the insides of the fluoroscopic images PI of the inside of the body of the patient P.

Here, a method in which the training image acquirer 111 provided in the learning device 110 acquires training images used for learning the first feature will be described. The training images include a plurality of images of a state in which the rod-shaped marker M to be tracked is photographed within a predetermined region (hereinafter referred to as "positive example images") and a plurality of images of the other states (hereinafter referred to as "negative example images"). Also, the other states in the negative example image are states, which are different from the state of the positive example image with respect to a state in which the rod-shaped marker M is photographed, for example, such as a state in which the rod-shaped marker M is not photographed within the predetermined region, a state in which the rod-shaped marker M is not photographed, and the like.

First, a method in which the training image acquirer 111 acquires a positive example image will be described. Here, an image, which is obtained by segmenting a region of a predetermined size (range) so that the center of gravity of the rod-shaped marker M is located at the center of an image from the entire region of the image captured by installing the target rod-shaped marker M to be tracked at a position between the radiation source 12 and the radiation detector 13 for capturing the first image which is the fluoroscopic image PI of the patient P while changing a posture of the rod-shaped marker M, is set as the positive example image. Also, as described above, because the rod-shaped marker M is made of a metallic substance, the marker image of the rod-shaped marker M is photographed as being dark in the captured image. Thus, the training image acquirer 111 extracts a center position of a region of pixels having small values (small pixel values) among pixels included in the captured image as the position of the center of gravity of the rod-shaped marker M.

The training image acquirer 111 may acquire a positive example image from images actually captured by the imaging device (the sets of the radiation sources 12 and the radiation detectors 13). Here, the image for acquiring the positive example image may be, for example, an image captured by an actual imaging device in a state in which an actual rod-shaped marker M is placed within a human phantom for photography that reproduces a human body. In this case, a positive example image when the radiation dose is different in the rod-shaped marker M of the same posture may be acquired by performing photography while a radiation dose (for example, an X-ray dose) of the radiation r radiated for photography is being changed. Also, an image for acquiring the positive example image is a fluoroscopic image PI obtained by the imaging device actually photographing the patient P in which the rod-shaped marker M is placed within the body of the patient P, i.e., a fluoroscopic image PI obtained by previously photographing the patient P.

Also, when a positive example image is acquired from images actually captured by the imaging device, it is not always possible to acquire (collect) positive example images associated with all available postures of the rod-shaped marker M placed within the body of the patient P. Thus, the training image acquirer 111 may be configured to construct an environment for reproducing (simulating) the posture of the rod-shaped marker M located between the radiation source 12 and the radiation detector 13, i.e., a simulation environment, using a computer or the like, and acquire a positive example image from simulation images which have been virtually created. Hereinafter, a method of acquiring a positive example image from simulation images virtually created by the constructed simulation environment will be described.

In the simulation environment constructed by the training image acquirer 111 to acquire a positive example image, the imaging device provided in the treatment device 10 constituting the treatment system 1 is first reproduced. More specifically, as shown in Fig. 1, positions of the radiation source 12 and the radiation detector 13 are fixed in the treatment device 10 constituting the treatment system 1. That is, in the treatment device 10, a photography direction of the imaging device including the set of the radiation source 12 and the radiation detector 13 is fixed. Thus, when predetermined three-dimensional coordinates are defined within the three-dimensional space in which the radiation source 12 and the radiation detector 13 are installed, the positions of the radiation source 12 and the radiation detector 13 can be represented by coordinate values of three axes. In the following description, information of the coordinate values of the three axes will be referred to as geometry information of the imaging device including the set of the radiation source 12 and the radiation detector 13.

The training image acquirer 111 reproduces the imaging device provided in the treatment device 10 using the geometry information. Also, information about a three-dimensional shape of the rod-shaped marker M (hereinafter referred to as "shape information") is known in advance. Thus, the training image acquirer 111 reproduces a three-dimensional space in which the rod-shaped marker M is placed between the radiation source 12-1 and the radiation detector 13-1 as shown in Fig. 5 using the geometry information and the shape information. Here, because it is possible to calculate attenuated energy of the radiation r-1 output from the radiation source 12-1 passing through the rod-shaped marker M in the three-dimensional space reproduced by the training image acquirer 111, the training image acquirer 111 can virtually acquire a marker image of the rod-shaped marker M photographed by the imaging device. At this time, the training image acquirer 111 does not need to precisely calculate the energy of the radiation r-1 which is attenuated while passing through the rod-shaped marker M. Accordingly, the training image acquirer 111 may virtually generate a simulation image in which pixels of the radiation detector 13-1 at which the radiation r-1 that has passed through the rod-shaped marker M and the radiation r-1 that has not passed through the rod-shaped marker M arrive are distinguished. At this time, the training image acquirer 111 may virtually generate a simulation image with a gradation in accordance with a length of a path along which the radiation r-1 has passed through the inside of the rod-shaped marker M. The training image acquirer 111 acquires a positive example image from simulation images generated in this manner.

Also, the treatment system 1 shown in Fig. 1 includes two imaging devices capable of simultaneously performing photography. Accordingly, the training image acquirer 111 virtually generates simulation images associated with the imaging devices and acquires a positive example image from the generated images. Here, a case in which the amount of rotation of the rod-shaped marker M around three axes set with respect to two axes orthogonal to an irradiation path of the radiation r-1 shown in Fig. 5 is set to $(\theta, \phi, \eta)$ is considered. In this case, the training image acquirer 111 uses three quantities of rotation of the rod-shaped marker M as parameters of the posture of the rod-shaped marker M (hereinafter referred to as "posture parameters") and acquires a positive example image from simulation images virtually generated while continuously changing each posture parameter. Also, the training image acquirer 111 may acquire a positive example image from simulation images virtually generated while changing the posture parameters at predetermined intervals such as, for example, 15° intervals, without continuously changing each posture parameter. Also, the training image acquirer 111 may acquire a positive example image from images obtained by combining the virtually generated simulation images with the fluoroscopic image PI or the clinical image of the patient P captured before the treatment according to the existing image compositing technology such as, for example, alpha blending, i.e., images reproduced together with noise and the like included in the fluoroscopic image PI or the clinical image. Also, a process of combining the simulation images and the fluoroscopic image PI or the clinical image is not limited to the use of the image compositing technology. For example, the training image acquirer 111 may combine the simulation image and the fluoroscopic image PI by replacing a pixel value of a pixel, which corresponds to the position of a rod-shaped marker M included in the simulation image, in the fluoroscopic image PI or the clinical image with a pixel value of the marker image or a predetermined pixel value (for example, pixel value="0" or the like).

Next, a method in which the training image acquirer 111 acquires a negative example image will be described. Here, an image obtained by segmenting a region having the same size as a region having a predetermined size in the positive example image from the entire region of the fluoroscopic image PI of the patient P captured before the treatment is set as a negative example image. That is, the negative example image is an image obtained by segmenting a region having the same size (range) as a predetermined region from the entire region of the fluoroscopic image PI so that the center of gravity of the rod-shaped marker M is located at the center of the image in the positive example image. Also, an image obtained by segmenting a region having the same size as that of the positive example image from an image of a human phantom or a clinical image obtained in a previous clinical period, i.e., an image actually captured by an imaging device, may be used as the negative example image. In this case, it is desirable that a radiation dose (for example, an X-ray dose) of the radiation r to be radiated when an image for obtaining a negative example image is captured be equivalent to a radiation dose (for example, an X-ray dose) of the radiation r radiated when an image for obtaining a positive example image is captured. Also, the negative example image may be an image in which a part or all of the rod-shaped marker M is photographed. In this regard, the positive example image and the negative example image have different regions in which the rod-shaped marker M is photographed. More specifically, in the positive example image, as described above, photography is performed within a region where the center of gravity of the rod-shaped marker M is located at the center of the positive example image. On the other hand, in the negative example image, the center of gravity of the rod-shaped marker M is photographed in a region other than a region that is the same as that of the positive example image.

The training image acquirer 111 acquires the above-described positive and negative example images as training images. The training image acquirer 111 outputs the acquired training images to the learner 112.

Thereby, the learner 112 learns a first feature common to marker images of the rod-shaped markers M on the basis of a plurality of training images output from the training image acquirer 111. The learner 112 includes a classifier of a function f that solves the two-class classification problem for identifying the positive example image and the negative example image output from the training image acquirer 111. The learner 112 uses any supervised learning classifier as the classifier included in the learner 112. More specifically, the learner 112 includes, for example, a two-class support vector machine, a neural network, a deep neural network, a convolutional neural network, a decision tree, or the like as the classifier. Also, an example of the classifier used by the learner 112 will be described together with an operation of the tracker 122 to be described below.

The learner 112 outputs a feature extraction parameter calculated from a first feature obtained through learning to the parameter storage 113. Thereby, the parameter storage 113 stores the feature extraction parameter output from the learner 112. The parameter storage 113 outputs the stored feature extraction parameter to the tracker 122 in response to a request for the feature extraction parameter output when the tracker 122 tracks the rod-shaped marker M.

In this manner, the learning device 110 acquires training images (a positive example image and a negative example image), learns a first feature common to the marker images of the rod-shaped marker M, calculates a feature extraction parameter, and causes the parameter storage 113 to store the calculated feature extraction parameter in the steps of the example of the flowchart showing the flow of the operation of the learning device 110 shown in Fig. 3.

Next, a marker tracking method in the moving object tracking device 120 constituting the medical image processing device 100 will be described. The moving object tracking device 120 tracks the rod-shaped marker M photographed in a first image (a fluoroscopic image PI) of the patient P captured during treatment.

Also, as shown in Fig. 1, in the treatment device 10 constituting the treatment system 1, the two imaging devices capture first images of the patient P in two directions. Thus, the first image acquirer 121 acquires the first images in the two directions captured by the two imaging devices, i.e., two fluoroscopic images PI.

Here, a method in which the first image acquirer 121 provided in the moving object tracking device 120 acquires a first image will be described. When radiation treatment is performed on the patient P, the patient P is first positioned so that the position of a lesion, bone, or the like within the body of the patient P is aligned with the position planned at the time of the treatment planning. More specifically, as shown in Fig. 1, a first image, which is the fluoroscopic image PI of the inside of the body of the patient P, is captured in a state in which the patient P is laid on the treatment table 11. Image collation is performed between the captured first image and a DRR image created from a three-dimensional CT image captured at the time of the treatment planning, the position deviation of the patient P between the images is obtained, and the position of a lesion or bone within the body of the patient P at present is aligned with the position planned at the time of the treatment planning by moving the treatment table 11 on the basis of the obtained position deviation of the patient. Thereafter, first images for several respiration processes are captured to confirm whether or not the rod-shaped marker M can be correctly tracked. At this time, the treatment beam B is not radiated to the patient P. After it is confirmed that the rod-shaped marker M can be correctly tracked, the radiation treatment is started. Also, each imaging device captures the first image at predetermined time intervals while radiation treatment is being performed. The first image acquirer 121 sequentially acquires first images captured by the imaging devices. The first image acquirer 121 outputs each of the acquired first images in the two directions to the tracker 122.

The tracker 122 tracks the rod-shaped marker M photographed in the first images in the two directions output from the first image acquirer 121. More specifically, the tracker 122 tracks the marker image of the rod-shaped marker M photographed in the first image output from the first image acquirer 121 on the basis of the first feature represented by the feature extraction parameter acquired from the parameter storage 113. Also, the tracker 122 uses geometry information of each imaging device provided in the treatment device 10 to obtain the position of the positioned rod-shaped marker M within the three-dimensional coordinates until the radiation r radiated from the radiation source 12 passes through the patient's body and arrives at the radiation detector 13. That is, the tracker 122 obtains the position of the rod-shaped marker M within the predetermined three-dimensional coordinates when the rod-shaped marker M is placed as shown in Fig. 5, as a projection matrix. Thus, the tracker 122 obtains the projection matrix associated with each imaging device from the geometry information in advance. That is, the tracker 122 obtains a projection matrix for each imaging device. The tracker 122 calculates coordinate values of three-dimensional coordinates representing the position of the rod-shaped marker M within a predetermined three-dimensional space using the principle of triangulation from positions (two-dimensional coordinate positions) of the rod-shaped markers M photographed in the two first images. Thereby, the tracker 122 calculates the position where the rod-shaped marker M within the predetermined three-dimensional space is photographed within the fluoroscopic image PI (the first image) of the inside of the body of the patient P.

Here, a method in which the tracker 122 provided in the moving object tracking device 120 tracks the rod-shaped marker M photographed in the first image will be described. When tracking of the rod-shaped marker M starts, the tracker 122 first predicts the position of the rod-shaped marker M within the first image output from the first image acquirer 121 in step S104 of the example of the flowchart showing the flow of the operation of the moving object tracking device 120 shown in Fig. 4.

As described above, the first image acquirer 121 sequentially acquires the first images captured by the imaging devices at predetermined time intervals even if the radiation treatment is being performed and outputs the first images to the tracker 122. Here, the position of the rod-shaped marker M is unknown in the initial stage when the tracker 122 has started a process of tracking the rod-shaped marker M with respect to the first image initially output from the first image acquirer 121. Thus, for example, the tracker 122 calculates positions of the rod-shaped marker M photographed in first images for several respiration processes captured after the alignment of the patient P is performed, i.e., first images before the radiation treatment starts (hereinafter referred to as "pre-treatment first images"), using the fact that the position of the rod-shaped marker M placed within the body of the patient P does not substantially move.

More specifically, in a process of predicting the position of the rod-shaped marker M in the initial stage when the process of tracking the rod-shaped marker M has been started, the tracker 122 projects the position of the rod-shaped marker M within the pre-treatment first image using the projection matrix obtained from the geometry information in advance. The tracker 122 designates a plurality of positions of the rod-shaped marker M within one pre-treatment first image (for example, a pre-treatment first image at the time of expiration or inspiration of the patient P) after the projection of the position of the rod-shaped marker M within each of the pre-treatment first images for one respiratory cycle is completed. Also, a region including the plurality of positions of the rod-shaped marker M designated in the pre-treatment first image is a region where the rod-shaped marker M is necessarily present. The tracker 122 sets a region where the rod-shaped marker M designated within the pre-treatment first image is necessarily present as the position (a predicted position) of the rod-shaped marker M predicted within the first image during the treatment.

Also, in the radiation treatment based on a respiratory synchronous irradiation method using the tracking of the marker, a four-dimensional CT image obtained by capturing a CT image as a moving image may be captured to make a treatment plan after the rod-shaped marker M is placed within the body of the patient P. The four-dimensional CT image is, for example, a moving image having a length of one respiratory cycle. In the four-dimensional CT image, the position of the rod-shaped marker M can be easily visually confirmed by a person (a doctor or the like) who carries out the radiation treatment using the treatment system 1. If the position (three-dimensional coordinates) of the rod-shaped marker M within the CT image is known, the position of the rod-shaped marker M can be projected within the pre-treatment first image using the projection matrix. Thus, the tracker 122 may set a region within the four-dimensional CT image designated by the user of the treatment system 1 as the region where the rod-shaped marker M is necessarily present. Also, the tracker 122 may set a region including a trajectory of the position of the rod-shaped marker M when the same rod-shaped marker M was tracked in the previous radiation treatment performed on the same lesion of the patient P as the region where the rod-shaped marker M is necessarily present in the current radiation treatment.

The tracker 122 predicts the position of the rod-shaped marker M photographed in the first image captured after the radiation treatment is started (hereinafter referred to as "the first image during the treatment") on the basis of the region where the rod-shaped marker M is necessarily present designated within the pre-treatment first image. Also, the position of the rod-shaped marker M is unknown because a stage when the region where the rod-shaped marker M is necessarily present is designated within the pre-treatment first image is the initial stage when the tracker 122 has started a process of tracking the rod-shaped marker M as described above. On the other hand, there is a high possibility that the rod-shaped marker M will be necessarily present within the designated region in the subsequent treatment stage. Also, in the treatment stage, first images during the treatment are output from the first image acquirer 121 at short intervals with respect to the respiratory cycle. Therefore, the tracker 122 predicts the position of the rod-shaped marker M in the treatment stage in a faster and simpler method.

More specifically, the tracker 122 sets a region (a range) centered on the region where the rod-shaped marker M is necessarily present predicted in the pre-treatment first image as a position (a predicted position) of the rod-shaped marker M predicted within the first image during the treatment in the process of predicting the position of the rod-shaped marker M in the treatment stage. Here, the predicted position of the rod-shaped marker M in the treatment stage may be a region of a part (a partial region) within the first image during the treatment or may be a space of a part (a partial space) in the three-dimensional space. That is, the predicted position of the rod-shaped marker M in the treatment stage may be two-dimensional coordinates or three-dimensional coordinates.

Also, in the process of predicting the position of the rod-shaped marker M in the treatment stage, the tracker 122 may set the position of the rod-shaped marker M as a predicted position of the rod-shaped marker M using a time-series filter for predicting a future position of the rod-shaped marker M from a sequence of previously obtained positions of the rod-shaped marker M. Here, for example, a Kalman filter, a particle filter, or the like can be considered as the time-series filter.

Subsequently, in step S105 of the example of the flowchart showing the flow of the operation of the moving object tracking device 120 shown in Fig. 4, the tracker 122 acquires the feature extraction parameter stored in the parameter storage 113 and calculates the likelihood of a predicted position of the rod-shaped marker M on the basis of a first feature represented by the acquired feature extraction parameter.

Here, the likelihood calculated by the tracker 122 is a value representing similarity of an image photographed at the position of the rod-shaped marker M within the first image during the treatment predicted by the tracker 122 to the marker image of the rod-shaped marker M. More specifically, the likelihood has a large value (the largest value in the case of the marker image) if the similarity to the marker image of the rod-shaped marker M is high and the likelihood has a small value if the similarity to the marker image of the rod-shaped marker M is low. The tracker 122 calculates the likelihood $l(x)$ of a predicted position $x$ of the rod-shaped marker M according to the following Eq. (1) or (2).

[Math. 1]

$$l(x) = f(v(x); w) \qquad \cdots (1)$$

[Math. 2]

$$l(x) = \begin{cases} f(v(x); w) & f(v(x); w) > Th \\ 0 & otherwise \end{cases} \qquad \cdots (2)$$

In the above Eqs. (1) and (2), the function f is a function representing a classifier that solves a two-class classification problem for identifying the positive example image and the negative example image output from the training image acquirer 111 included in the learner 112. That is, the tracker 122 includes a classifier having a function f similar to that of the classifier included in the learner 112. Also, in the above Eqs. (1) and (2), $v(x)$ is a vector in which pixel values of the first image during the treatment determined on the basis of a predicted position $x$ of the rod-shaped marker M are arranged. Also, $v(x)$ may be a vector obtained by converting the vector in which the pixel values of the first image during the treatment determined on the basis of the predicted position $x$ of the rod-shaped marker M are arranged. The conversion in this case is, for example, conversion by a differential filter such as a Sobel filter. Also, in the above Eqs. (1) and (2), w is a parameter constituting the classifier of the function f and is a feature extraction parameter representing the first feature, i.e., a feature extraction parameter acquired from the parameter storage 113.

Also, as shown in Fig. 1, in the treatment device 10 constituting the treatment system 1, two first images (fluoroscopic images PI) during the treatment in which the same rod-shaped marker M is photographed in two different directions at the same time are generated. Thus, the tracker 122 can calculate the likelihood $l(x)$ associated with each first image during the treatment from the predicted position of the rod-shaped marker M in each first image during the treatment according to the above Eq. (1) or the above Eq. (2). At this time, when the predicted position of the rod-shaped marker M in the treatment stage has three-dimensional coordinates, the tracker 122 may calculate likelihoods $l(x)$ associated with the two first images during the treatment captured at the same time and set a product of the calculated likelihoods $l(x)$ as the likelihood $l(x)$ of the predicted position of the rod-shaped marker M. On the other hand, when the predicted position of the rod-shaped marker M in the treatment stage has two-dimensional coordinates, it is only necessary for the tracker 122 to calculate likelihoods $l(x)$ for predicted positions of two first images during the treatment for which an epipolar constraint is satisfied.

Also, the tracker 122 may set the vector $v(x)$ in which the pixel values determined on the basis of the predicted positions $x$ of the rod-shaped marker M in the two first images during the treatment simultaneously generated in the treatment device 10 are arranged as a connected vector. In this case, the predicted positions are two-dimensional coordinate positions where the predicted position of the rod-shaped marker M is projected onto the two first images during the treatment. Also, in this case, the two-dimensional coordinate positions are predicted positions of the two first images during the treatment for which the epipolar constraint is satisfied.

Subsequently, in step S106 of the example of the flowchart showing the flow of the operation of the moving object tracking device 120 shown in Fig. 4, the tracker 122 calculates the position of the rod-shaped marker M included in the first image during the treatment on the basis of the calculated likelihood l(x).

The tracker 122 calculates a position $x_m$ of the rod-shaped marker M according to the following Eq. (3) on the basis of the calculated likelihood l(x).

[Math. 3]

$$ x_m = \sum_{x \in S} l(x) x \qquad \cdots (3) $$

As can be seen from the above Eq. (3), the tracker 122 sets a weighted average in which the likelihood l(x) is set as a weighted value as the position $x_m$ of the rod-shaped marker M. Also, in the above Eq. (3), S denotes a set of predicted positions of the rod-shaped marker M.

The tracker 122 may calculate the position $x_m$ of the rod-shaped marker M according to the following Eq. (4) on the basis of the calculated likelihood l(x).

[Math. 4]

$$ x_m = \arg\max_{x \in S} l(x) \qquad \cdots (4) $$

In the above Eq. (4), the tracker 122 sets a predicted position where the likelihood l(x) is a maximum as the position $x_m$ of the rod-shaped marker M.

In this manner, in the steps of the example of the flowchart showing the flow of the operation of the moving object tracking device 120 shown in Fig. 4, the moving object tracking device 120 predicts the position of the rod-shaped marker M within the first image during the treatment, calculates the likelihood l(x) of the predicted position of the rod-shaped marker M based on the first feature represented by the feature extraction parameter, and calculates the position $x_m$ of the rod-shaped marker M included in the first image during the treatment. The moving object tracking device 120 outputs a marker position signal SM representing the calculated position $x_m$ of the rod-shaped marker M.

As described above, in the medical image processing device 100 of the first embodiment, the learning device 110 (more specifically, the training image acquirer 111) acquires simulation images obtained by performing simulation in all available directions of the rod-shaped marker M when the rod-shaped marker M is placed within the body of the patient P as training images. In the medical image processing device 100 of the first embodiment, the learning device 110 (more specifically, the learner 112) learns a first feature common to the rod-shaped markers M photographed in training images from a plurality of training images that have been acquired, calculates a feature extraction parameter representing a feature of a direction of the rod-shaped marker M on the basis of the first feature obtained through learning, and causes the parameter storage 113 to store the feature extraction parameter. In the medical image processing device 100 of the first embodiment, the moving object tracking device 120 (more specifically, the first image acquirer 121) acquires the first image (the fluoroscopic image PI) of the patient P captured during the treatment. In the medical image processing device 100 of the first embodiment, the moving object tracking device 120 (more specifically, the tracker 122) calculates (extracts) a feature (a "first feature" in the first embodiment) of the first image similar to a first feature represented by the feature extraction parameter acquired from the parameter storage 113. In the medical image processing device 100 of the first embodiment, the moving object tracking device 120 (more specifically, the tracker 122) tracks the rod-shaped marker M photographed in the first image on the basis of the calculated first feature and outputs a marker position signal SM representing the position of the rod-shaped marker M.

Moreover, in the medical image processing device 100 of the first embodiment, the rod-shaped marker M is not tracked according to template matching using a template as in the conventional technology. Thus, the medical image processing device 100 of the first embodiment can efficiently perform the calculation when the rod-shaped marker M is tracked and curb the deterioration of real-time performance of the tracking of the rod-shaped marker M. Also, the medical image processing device 100 of the first embodiment can reduce a possibility of erroneous tracking of the rod-shaped marker M that may be caused due to the presence of a template having a high degree of similarity to the rod-shaped marker M in the conventional template matching.

Thereby, the treatment system 1 including the medical image processing device 100 of the first embodiment can automatically detect an appropriate timing at which a moving lesion will be irradiated with the treatment beam B in conjunction with the respiration or heartbeat of the patient P or the like on the basis of the position of the rod-shaped marker M tracked within the body of the patient P. Thereby, the treatment system 1 including the medical image processing device 100 of the first embodiment can safely irradiate the lesion with the treatment beam B at an appropriate timing synchronized with the expiration or inspiration of the patient P.

Also, in the medical image processing device 100 of the first embodiment, a case in which the target marker to be tracked is the rod-shaped marker M has been described. However, as described above, the target marker to be tracked has various shapes. Accordingly, the target marker to be tracked by the medical image processing device 100 of the first embodiment is not limited to the rod-shaped marker M. The medical image processing device 100 of the first embodiment can perform a similar operation (processing) by acquiring simulation images obtained through simulation in all available directions when each marker is placed within the body of the patient P as training images on the basis of a similar concept even if the target marker to be tracked is different from the rod-shaped marker M. That is, the medical image processing device 100 of the first embodiment can track the position of each marker in a manner similar to that when the position of the rod-shaped marker M is tracked by learning simulation images, which are acquired as in an example of a relationship between a radiation irradiation path and a marker (a rod-shaped marker) shown in Fig. 5, as training images even if the target marker to be tracked is, for example, a wedge-shaped marker or a clip-shaped marker or further is a spherical marker. Also, in the tracking performed by the medical image processing device 100 of the first embodiment, it is possible to track an image of a catheter shown in a fluoroscopic image PI of the patient captured during a surgical operation, for example, by learning a simulation image of the catheter as a training image, as well as the marker.

As described above, the medical image processing device 100 includes the first image acquirer 121 configured to acquire the fluoroscopic image PI of the patient P as a first image; and the tracker 122 configured to track an object (a marker) photographed in the first image on the basis of a first feature common to object images (marker images) that are a plurality of images of the object obtained by observing the object placed within a body of the patient P in a plurality of directions.

Also, as described above, the medical image processing device 100 may further include the training image acquirer 111 configured to acquire a plurality of training images associated with the plurality of object images; and the learner 112 configured to learn the first feature common to the object images included in the plurality of training images.

Also, as described above, the training image acquirer 111 may acquire the training image based on a simulation image obtained by simulating the object image when the object is photographed in the first image on the basis of geometry information of an imaging device (the set of the radiation source 12 and the radiation detector 13) that captures the first image and a three-dimensional shape of the object.

Also, as described above, the training image may be an image in which the simulation image and the first image are combined.

Also, as described above, the training image may be an image in which the simulation image and a clinical image obtained by photographing a range, which is the same as that of the first image, are combined.

Also, as described above, the training image may include a positive example image of a range that is predetermined so that a center of gravity of the object image is located at a center thereof and a negative example image in which the center of gravity of the object image is different from that in a state of the positive example image and which has the same range as the positive example image.

Also, as described above, the object may be a marker (for example, the rod-shaped marker M) placed within the body of the patient P.

Also, as described above, the marker may have a shape other than a spherical shape (for example, a rod shape).

Also, as described above, the treatment system 1 may include the medical image processing device 100; the treatment device 10 including an irradiator (the treatment beam irradiation gate 14) configured to irradiate a treatment target portion (a lesion) with a treatment beam B and an imaging device (the set of the radiation source 12 and the radiation detector 13) configured to photograph the object; and a controller configured to control the treatment on the lesion on the basis of position information of the tracked object.

Also, the medical image processing device 100 includes a processor such as a CPU or a GPU and a storage device such as a ROM, a RAM, an HDD, or a flash memory. The storage device may be a device storing a program for causing the processor to function as: the first image acquirer 121 configured to acquire the fluoroscopic image PI of the patient P as a first image; and the tracker 122 configured to track an object (a marker) photographed in the first image on the basis of a first feature common to object images (marker images) that are a plurality of images of the object obtained by observing the object placed within a body of the patient P in a plurality of directions.

Also, the medical image processing device 100 includes a processor such as a CPU or a GPU and a storage device such as a ROM, a RAM, an HDD, or a flash memory. The storage device may be a device storing a program for causing the processor to function as: the training image acquirer 111 configured to acquire a plurality of training images associated with the plurality of object images; and the learner 112 configured to learn the first feature common to the object images included in the plurality of training images.

(Second embodiment)

Hereinafter, a second embodiment will be described. The configuration of a treatment system including a medical image processing device of the second embodiment is a configuration in which the medical image processing device 100 in the configuration of the treatment system 1 including the medical image processing device 100 of the first embodiment shown in Fig. 1 is replaced with a medical image processing device of the second embodiment (hereinafter referred to as a "medical image processing device 200"). In the following description, the treatment system including the medical image processing device 200 is referred to as a "treatment system 2."

Also, in the following description, the components of the treatment system 2 including the medical image processing device 200 similar to those of the treatment system 1 including the medical image processing device 100 of the first embodiment are denoted by the same reference signs and a detailed description of similar components will be omitted. In the following description, only a configuration, an operation, and a process of the medical image processing device 200, which is a component different from the medical image processing device 100 of the first embodiment, will be described.

The medical image processing device 200 tracks a marker placed within a body of a patient P to be treated in radiation treatment on the basis of fluoroscopic images PI output from a radiation detector 13-1 and a radiation detector 13-2 as in the medical image processing device 100 of the first embodiment. Thereby, in the medical image processing device 200, as in the medical image processing device 100 of the first embodiment, it is also possible to track a lesion within the body of the patient P to be treated in the radiation treatment. Also, the medical image processing device 200 limits a marker image to be learned by learning a marker image of a placed marker, or estimating and learning a posture of the placed marker, from the fluoroscopic image PI of the patient P captured before the treatment. Here, the marker image to be learned in the medical image processing device 200 is limited because, even if the marker actually placed in the patient P moves within the body in conjunction with the respiration or heartbeat of the patient P or the like, it is considered that the posture of the marker is unlikely to be significantly changed from a posture when the marker has been placed. The medical image processing device 200 automatically detects a timing at which the lesion will be irradiated with the treatment beam B in the radiation treatment on the basis of a result of tracking the marker placed within the body of the patient P as in the medical image processing device 100 of the first embodiment.

Hereinafter, the configuration of the medical image processing device 200 constituting the treatment system 2 will be described. Fig. 6 is a block diagram showing a schematic configuration of the medical image processing device 200 of the second embodiment. The medical image processing device 200 shown in Fig. 6 includes a learning device 210 and a moving object tracking device 120. Also, the learning device 210 includes a training image acquirer 211, a learner 112, a parameter storage 113, a second image acquirer 214, and an object extractor 215. Also, the moving object tracking device 120 includes a first image acquirer 121 and a tracker 122.

The medical image processing device 200 has a configuration in which the learning device 110 constituting the medical image processing device 100 of the first embodiment is replaced with the learning device 210. The learning device 210 has a configuration in which the second image acquirer 214 and the object extractor 215 are added to the learning device 110 constituting the medical image processing device 100 of the first embodiment. In association with this, the medical image processing device 200 has the training image acquirer 211 with which the training image acquirer 111 provided in the learning device 110 constituting the medical image processing device 100 of the first embodiment is replaced. The other components provided in the medical image processing device 200 are the same as those provided in the medical image processing device 100 of the first embodiment. Accordingly, in the following description, the components of the medical image processing device 200 similar to those provided in the medical image processing device 100 of the first embodiment are denoted by the same reference signs and a detailed description of similar components will be omitted. In the following description, only components different from those of the medical image processing device 100 of the first embodiment will be described.

The learning device 210 acquires training images in the medical image processing device 200 before radiation treatment is performed, learns a first feature for detecting a marker image from the acquired training images, calculates a feature extraction parameter representing a feature of a marker on the basis of the learned first feature, and causes the parameter storage 113 to store the feature extraction parameter as in the learning device 110 constituting the medical image processing device 100 of the first embodiment. At this time, the learning device 210 limits the marker image for learning the first feature on the basis of the fluoroscopic image PI of the patient P captured before the treatment.

The second image acquirer 214 acquires a second image which is a fluoroscopic image PI of the patient P captured before the treatment. The second image acquirer 214 outputs the acquired second image to the object extractor 215. Here, the second image is the fluoroscopic image PI of the patient P before the treatment after the patient P is laid and aligned on the treatment table 11 in a state in which the marker is placed within the body of the patient P. That is, the second image is the fluoroscopic image PI generated by the radiation detector 13 in accordance with radiation r radiated by the radiation source 12 before the radiation treatment is performed (in a state in which the treatment beam irradiation gate 14 does not radiate the treatment beam B). Also, the second image acquirer 214 may include an interface for connecting to the radiation detector 13 provided in the treatment device 10.

Also, it is only necessary for the second image to be a fluoroscopic image PI of the patient P before the treatment in a state in which the marker is placed within the body of the patient P. Thus, it is only necessary for the second image to be a fluoroscopic image PI of the patient P captured in a treatment planning stage or a stage before the treatment planning stage when radiation treatment is performed. For example, a DRR image created from a three-dimensional CT image captured at the time of the treatment planning may be acquired as the second image. Also, because treatment is normally performed a plurality of times in the radiation treatment, for example, the first image captured in the previous radiation treatment performed on the same lesion of the patient P may be acquired as the second image. Also, in the radiation treatment based on a respiratory synchronous irradiation method using the tracking of the marker, first images for several respiration processes may be captured as a rehearsal for radiating the treatment beam B in synchronization with the respiration of the patient P. In this case, three first images captured at the time of this rehearsal may be acquired as second images. Also, when CT photography is performed in a state in which the patient P is laid on the treatment table 11 in the treatment system 2 before the radiation treatment is started, a DRR image created from a three-dimensional CT image captured at that time may be acquired as the second image.

The object extractor 215 acquires the second image from the second image acquirer 214 and extracts a marker image included as an object image within the acquired second image. The object extractor 215 outputs an image of a region of a predetermined part (a partial region) including the extracted marker image or information (an image) of a result of estimating the posture of the marker from the image of the partial region including the extracted marker image to the training image acquirer 211. Here, the extraction of the marker in the object extractor 215 can be performed according to a process similar to that of the learner 112 provided in the learning device 110 constituting the medical image processing device 100 of the first embodiment or the classifier provided in the tracker 122 provided in the moving object tracking device 120. In this regard, the second image used by the object extractor 215 for extracting the marker image is an image including the marker placed within the body of the patient P captured before the treatment is started. Accordingly, although the moving object tracking device 120 is provided as a configuration in which the medical image processing device 100 tracks a marker of any posture in real time in the first embodiment, real-time performance is not required in the extraction of the marker by the object extractor 215. Thus, the object extractor 215 may extract the marker from the second image according to, for example, the existing template matching. In this case, the object extractor 215 may output a template image extracted according to the template matching as an image of the partial region including the marker image to the training image acquirer 211. Also, the object extractor 215 may output a posture parameter associated with the posture of the marker represented by the template image extracted according to the template matching as information of a result of estimating the posture of the marker to the training image acquirer 211.

Also, the object extractor 215 may output an image of a partial region where the marker image is not located within a predetermined range at the center of the image as a negative example image to the training image acquirer 211 when an image obtained by segmenting a partial region having a predetermined size (range) so that the center of gravity of the marker segmented from the second image is located at the center of the image is output as a positive example image of a partial region including a marker image to the training image acquirer 211.

The training image acquirer 211 acquires a training image in the medical image processing device 200 and outputs the acquired training image to the learner 112 as in the training image acquirer 111 provided in the learning device 110 constituting the medical image processing device 100 of the first embodiment. Also, the training image acquirer 211 acquires an image of a partial region (which may also be an image of a template) including the marker image output from the object extractor 215. In this case, the training image acquirer 211 outputs training images (a positive example image and a negative example image) in which the image of the partial region including the marker image output from the object extractor 215 is combined with the clinical image to the learner 112 instead of a simulation image in the training image acquirer 111 provided in the learning device 110 constituting the medical image processing device 100 of the first embodiment. Also, when the object extractor 215 outputs the information of the result of estimating the posture of the marker from the image of the partial region including the marker image, the training image acquirer 211 acquires information (a posture parameter) of the result of estimating the posture of the marker output from the object extractor 215. In this case, the training image acquirer 211 outputs training images (a positive example image and a negative example image) in which a simulation image associated with the information (the posture parameter) of the result of estimating the posture of the marker output from the object extractor 215 is combined with the clinical image to the learner 112.

Also, when the object extractor 215 outputs a positive example image of a partial region including a marker image, the training image acquirer 211 acquires an associated negative example image and outputs the positive example image output from the object extractor 215 and the acquired negative example image as training images to the learner 112. Also, when the object extractor 215 outputs the positive example image and the negative example image as the images of the partial regions including the marker images, the training image acquirer 211 outputs training images including the positive example image and the negative example image output from the object extractor 215 to the learner 112.

Also, the function of acquiring the image of the partial region including the marker image or the information of the result of estimating the posture of the marker output from the object extractor 215 in the training image acquirer 211 and outputting the training image to the learner 112 may be a function added to the function of the training image acquirer 111 provided in the learning device 110 constituting the medical image processing device 100 of the first embodiment or may be a function instead of the function of the training image acquirer 111.

According to this configuration, the medical image processing device 200 limits a marker image to be learned on the basis of a concept that a posture of the marker actually placed in the patient P is unlikely to be significantly changed in conjunction with the respiration or heartbeat of the patient P or the like. More specifically, the medical image processing device 200 extracts the marker image of the placed marker from a fluoroscopic image PI of the patient P captured before the treatment or estimates a posture of the placed marker. As in the medical image processing device 100 of the first embodiment, the medical image processing device 200 learns the first feature common to the marker images on the basis of the extracted marker image or the estimated posture of the marker image, i.e., the training image associated with the limited marker image. Thereby, the medical image processing device 200 can limit the marker image to be learned as compared with the medical image processing device 100 of the first embodiment that performs learning associated with the marker in any posture. In the medical image processing device 200, as in the medical image processing device 100 of the first embodiment, the marker photographed in the first image which is the fluoroscopic image PI of the patient P captured during the treatment is tracked on the basis of the learned feature of the marker and a marker position signal SM representing the position of the tracked marker is output. Moreover, the medical image processing device 200 can improve the accuracy in tracking the marker because the target marker to be tracked is limited to the marker within the body of the patient P at present. Thereby, in the medical image processing device 200, as in the medical image processing device 100 of the first embodiment, it is also possible to automatically detect a timing at which the lesion within the body of the patient P will be irradiated with the treatment beam B. Thereby, in the treatment system 2 including the medical image processing device 200, as in the treatment system 1 including the medical image processing device 100 of the first embodiment, it is also possible to irradiate a lesion within the body of the patient P with the treatment beam B at an appropriate timing.

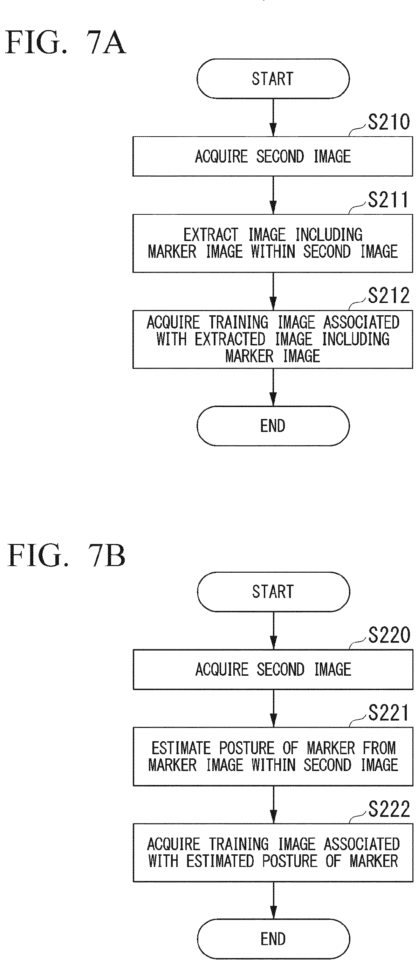

Here, the operation of the medical image processing device 200 constituting the treatment system 2 will be schematically described. Here, the operation of the learning device 210 constituting the medical image processing device 200 will be schematically described. Figs. 7A and 7B are examples of a flowchart showing a flow of an operation of the learning device 210 constituting the medical image processing device 200 of the second embodiment. In Fig. 7A, an example of the flow of the operation when the learning device 210 acquires a training image associated with a marker image extracted from a second image is shown. Also, in Fig. 7B, an example of the flow of the operation when the learning device 210 acquires a training image associated with a posture of the marker estimated from the marker image extracted from the second image is shown. The learning device 210 provided in the medical image processing device 200 performs one or both of the operation shown in Fig. 7A and the operation shown in Fig. 7B to acquire a training image. Also, in the example of the flowchart showing the flow of the operation of the learning device 210 shown in Fig. 7, the flow of an operation of learning the first feature and storing the feature extraction parameter in the parameter storage 113 similar to that of the learning device 110 provided in the medical image processing device 100 of the first embodiment to be performed after the training image is acquired will be omitted.

First, an operation in which the learning device 210 acquires a training image associated with a marker image extracted from a second image will be described with reference to Fig. 7A. When the learning device 210 starts the operation, the second image acquirer 214 first acquires the second image (step S210). Subsequently, the object extractor 215 extracts the marker image included within the second image output from the second image acquirer 214 (step S211). Subsequently, the training image acquirer 211 acquires a training image associated with an image of a partial region (which may also be an image of a template) including the marker image output from the object extractor 215 (step S212). Subsequently, the learning device 210 acquires training images (a positive example image and a negative example image), learns a first feature common to marker images of extracted markers, calculates a feature extraction parameter, outputs the calculated feature extraction parameter to the parameter storage 113, and causes the parameter storage 113 to store the calculated feature extraction parameter as in the steps of the example of the flowchart showing the flow of the operation of the learning device 110 provided in the medical image processing device 100 of the first embodiment shown in Fig. 3.

Next, an operation in which the learning device 210 acquires a training image associated with a posture of a marker estimated from a marker image extracted from a second image will be described with reference to Fig. 7B. When the learning device 210 starts the operation, the second image acquirer 214 first acquires the second image (step S220). Subsequently, the object extractor 215 extracts a marker image included within the second image output from the second image acquirer 214 and estimates the posture of the marker from an image of a partial region (which may also be an image of a template) including the extracted marker image (step S221). Subsequently, the training image acquirer 211 acquires a training image associated with information (a posture parameter) of a result of estimating the posture of the marker output from the object extractor 215 (step S222). Subsequently, the learning device 210 acquires training images (a positive example image and a negative example image), learns a first feature common to marker images of extracted markers, calculates a feature extraction parameter, outputs the calculated feature extraction parameter to the parameter storage 113, and causes the parameter storage 113 to store the calculated feature extraction parameter as in the steps of the example of the flowchart showing the flow of the operation of the learning device 110 provided in the medical image processing device 100 of the first embodiment shown in Fig. 3.

Next, details of the operation of the learning device 210 provided in the medical image processing device 200 constituting the treatment system 2 will be described. First, a method in which the object extractor 215 provided in the learning device 210 constituting the medical image processing device 200 extracts a marker image included within a second image in step S211 or step S221 of the example of the flowchart showing the flow of the operation of the learning device 210 shown in Fig. 7 will be described. Also, because the extraction of the marker image from the second image by the object extractor 215 is performed in the step before the training image is acquired, real-time performance is not required as described above.

Because the visibility of the marker is high when the second image is a three-dimensional CT image captured at the time of the treatment planning, a radiation treatment provider (a doctor or the like) using the treatment system 2 can designate a marker image while performing visual confirmation. In this case, the object extractor 215 can easily extract the marker image within the CT image designated by the user of the treatment system 1 as the marker image included within the second image. The object extractor 215 can segment a region of a predetermined part (a partial region) including the marker image extracted from the CT image and output the segmented region as an image of the partial region including the marker image. Also, in this case, the object extractor 215 can easily estimate the posture of the marker on the basis of the information of the marker image within the CT image designated by the user of the treatment system 1. Because the CT image is a three-dimensional image, the object extractor 215 may segment a region of a part including the marker image within the twodimensional DRR image created from the CT image on the basis of the information of the marker image within the designated CT image and output the segmented region as an image of the partial region including the marker image.

Also, when the second image is a fluoroscopic image PI, it is possible to identify the position of a marker according to a process similar to that of the learner 112 provided in the learning device 110 constituting the medical image processing device 100 of the first embodiment or the classifier provided in the tracker 122 provided in the moving object tracking device 120. In this case, the object extractor 215 can extract a marker image included within the second image on the basis of the identified position information of the marker. Also, in this case, the object extractor 215 can estimate the posture of the marker from the marker image displayed at the identified position of the marker.

Also, as described above, the object extractor 215 can identify the position of the marker included within the second image according to template matching. In this case, the object extractor 215 can extract the marker image included within the second image on the basis of the position information of the marker identified according to the template matching. Also, in this case, the object extractor 215 can easily estimate the posture of the marker on the basis of the information of the marker image within the CT image designated by the user of the treatment system 1. Also, in this case, the object extractor 215 can estimate the posture of the marker from the template used when the marker is identified.

Here, a method in which the object extractor 215 provided in the learning device 210 constituting the medical image processing device 200 estimates a posture of a marker from an extracted marker image in step S221 of the example of the flowchart showing the flow of the operation of the learning device 210 shown in Fig. 7B will be described. Here, a method in which the object extractor 215 estimates a posture of a marker when a marker image included within a second image has been extracted according to template matching will be described. Also, a case in which a marker placed within the body of the patient P is a marker other than a spherical marker and a posture of a rod-shaped marker M is estimated as an example will be described below.

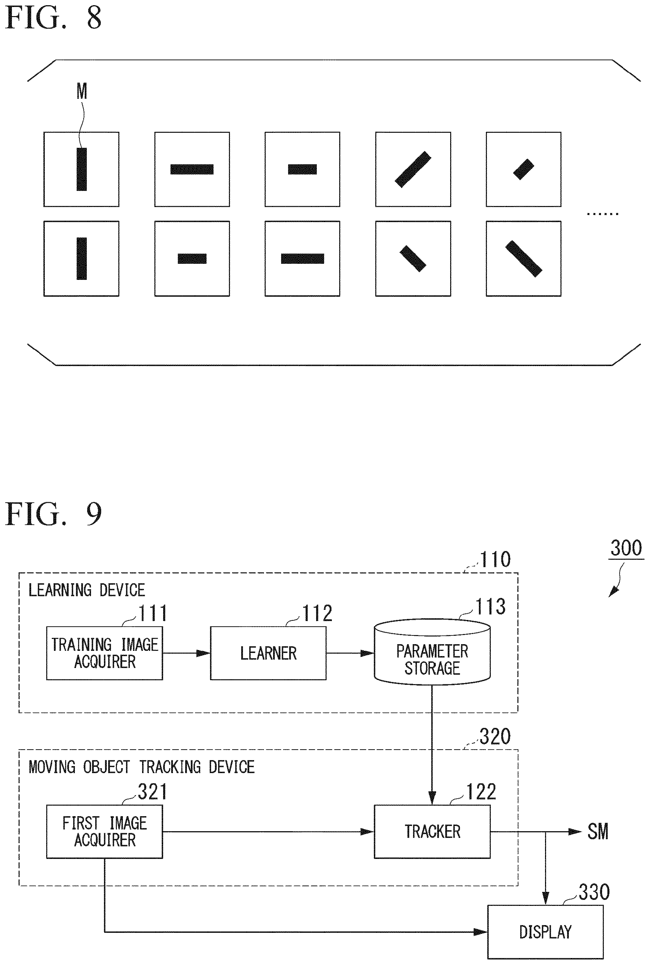

Fig. 8 is a diagram of an example of a marker image (a marker image of a rod-shaped marker M) extracted by the learning device 210 constituting the medical image processing device 200 of the second embodiment. The marker image of the rod-shaped marker M shown in Fig. 8 may also be a virtually created simulation image acquired by the training image acquirer 111 provided in the learning device 110 constituting the medical image processing device 100 of the first embodiment. Also, the marker image of the rod-shaped marker M shown in Fig. 8 can also be used as an image of a template used when the object extractor 215 extracts the marker image of the rod-shaped marker M according to template matching. In Fig. 8, a set of marker images of the rod-shaped marker M capable of being photographed in two directions at the same time by the two imaging devices provided in the treatment system 2 is shown. In Fig. 8, an upper marker image and a lower marker image in each column are one set in each posture of the rod-shaped marker M.

Because the marker images of the rod-shaped marker M shown in Fig. 8 are those created while the posture parameter representing the posture of the rod-shaped marker M is being sequentially changed, the marker image of the rod-shaped marker M corresponds to the posture parameter of the rod-shaped marker M. Thus, the object extractor 215 can use each set of marker images of the rod-shaped marker M shown in Fig. 8 as a template and calculate the degree of similarity between each template set and the marker image of the rod-shaped marker M photographed in each second image. Here, it is possible to use normalized cross-correlation, the amount of mutual information, or the like for the calculation of the degree of similarity in the object extractor 215. The object extractor 215 can estimate the posture of the rod-shaped marker M photographed in the second image by identifying the posture parameter associated with the template having the highest degree of similarity.

In this manner, the object extractor 215 extracts the marker image of the placed rod-shaped marker M from the fluoroscopic image PI of the patient P captured before the treatment and outputs an image of a partial region including the extracted marker image of the rod-shaped marker M or information (an image) of a result of estimating the posture of the marker from the image of the partial region including the extracted marker image to the training image acquirer 211. Subsequently, in the medical image processing device 200, as in the learning device 110 included in the medical image processing device 100 of the first embodiment, the learning device 210 also acquires training images (a positive example image and a negative example image), learns a first feature common to marker images of the rod-shaped marker M, calculates a feature extraction parameter, and causes the parameter storage 113 to store the calculated feature extraction parameter. In the medical image processing device 200, the moving object tracking device 120 also predicts the position of the rod-shaped marker M within the first image during the treatment, calculates the likelihood l(x) of the predicted position of the rod-shaped marker M based on the first feature represented by the feature extraction parameter, and calculates a position $x_m$ of the rod-shaped marker M included in the first image during the treatment. In the medical image processing device 200, the moving object tracking device 120 also outputs the marker position signal SM representing the calculated position $x_m$ of the rod-shaped marker M.

As described above, in the medical image processing device 200 of the second embodiment, the learning device 210 (more specifically, the object extractor 215) extracts a marker image of the placed rod-shaped marker M from a fluoroscopic image PI of the patient P captured before the treatment or estimates a posture of the placed rod-shaped marker M on the basis of a concept that the posture of the marker actually placed in the patient P is unlikely to be significantly changed in conjunction with the respiration or heartbeat of the patient P or the like. Thereby, in the medical image processing device 200 of the second embodiment, it is possible to limit the marker image of the rod-shaped marker M to be learned. In the medical image processing device 200 of the second embodiment, the learning device 210 (more specifically, the training image acquirer 211) learns a first feature common to marker images of the rod-shaped marker M having a limited posture placed within the body of the patient P on the basis of the extracted marker image or the estimated posture of the marker image, i.e., a training image associated with the limited marker image of the rod-shaped marker M. Subsequently, in the medical image processing device 200 of the second embodiment, as in the medical image processing device 100 of the first embodiment, a feature extraction parameter representing a feature of a direction of the rod-shaped marker M is calculated on the basis of a first feature obtained through learning and stored in the parameter storage 113. In the medical image processing device 200 of the second embodiment, as in the medical image processing device 100 of the first embodiment, the moving object tracking device 120 tracks the rod-shaped marker M photographed in the first image (the fluoroscopic image PI) of the patient P captured during the treatment and outputs a marker position signal SM representing the position of the rod-shaped marker M.

Thereby, in the medical image processing device 200 of the second embodiment, as in the medical image processing device 100 of the first embodiment, it is also possible to efficiently perform calculation when the rod-shaped marker M is tracked and curb deterioration of the real-time performance of tracking of the rod-shaped marker M. Also, in the medical image processing device 200 of the second embodiment, as in the medical image processing device 100 of the first embodiment, it is also possible to reduce the possibility of erroneous tracking of the rod-shaped marker M that may be caused due to the presence of a template having a high degree of similarity to the rod-shaped marker M in the conventional template matching.

Thereby, in the treatment system 2 including the medical image processing device 200 of the second embodiment, as in the treatment system 1 including the medical image processing device 100 of the first embodiment, it is also possible to safely irradiate the lesion with the treatment beam B at an appropriate timing synchronized with the expiration or inspiration of the patient P.

Moreover, the medical image processing device 200 of the second embodiment can improve the accuracy when the rod-shaped marker M is tracked because the rod-shaped marker M to be tracked is limited to the rod-shaped marker M in the posture within the body of the patient P at present.

Also, although a case in which the target marker to be tracked is the rod-shaped marker M has been described in the medical image processing device 200 of the second embodiment, a target marker to be tracked is not limited to the rod-shaped marker M as in the medical image processing device 100 of the first embodiment.

As described above, the medical image processing device 200 further includes the second image acquirer 214 configured to acquire a fluoroscopic image PI captured at a time different from that of a first image as a second image; and the object extractor 215 configured to extract an object image (a marker) photographed in the second image, wherein the training image acquirer 211 acquires a training image associated with a posture of an object photographed in the second image.

Also, as described above, the training image may be an image in which the object image extracted from the second image and a clinical image in which a range, which is the same as that of the second image, is photographed are combined.

(Third embodiment)

A third embodiment will be described below. Also, a configuration of a treatment system including a medical image processing device of the third embodiment is a configuration in which the medical image processing device 100 in the configuration of the treatment system 1 including the medical image processing device 100 of the first embodiment shown in Fig. 1 is replaced with the medical image processing device of the third embodiment (hereinafter referred to as a "medical image processing device 300"). In the following description, the treatment system including the medical image processing device 300 is referred to as a "treatment system 3."

Also, in the following description, the components of the treatment system 3 including the medical image processing device 300 similar to those of the treatment system 1 including the medical image processing device 100 of the first embodiment are denoted by the same reference signs and a detailed description of similar components will be omitted. In the following description, only a configuration, an operation, and a process of the medical image processing device 300, which is a component different from the medical image processing device 100 of the first embodiment, will be described.

The medical image processing device 300 tracks a marker placed within a body of a patient P to be treated in radiation treatment on the basis of fluoroscopic images PI output from a radiation detector 13-1 and a radiation detector 13-2 as in the medical image processing device 100 of the first embodiment. Thereby, in the medical image processing device 300, as in the medical image processing device 100 of the first embodiment, it is also possible to track a lesion within the body of the patient P to be treated in the radiation treatment. The medical image processing device 300 automatically detects a timing at which the lesion will be irradiated with the treatment beam B in the radiation treatment on the basis of a result of tracking the marker placed within the body of the patient P as in the medical image processing device 100 of the first embodiment. Also, the medical image processing device 300 allows a radiation treatment provider (a doctor or the like) using the treatment system 3 to perform monitoring by displaying a state in which a marker placed within the body of the patient P is being tracked. Also, the medical image processing device 300 allows the radiation treatment provider (the doctor or the like) using the treatment system 3 to confirm that treatment is being performed normally by further displaying the position of the lesion and the like estimated from the position of the marker which is being tracked.

The configuration of the medical image processing device 300 constituting the treatment system 3 will be described below. Fig. 9 is a block diagram showing a schematic configuration of the medical image processing device 300 of the third embodiment. The medical image processing device 300 shown in Fig. 9 includes a learning device 110, a moving object tracking device 320, and a display 330. Also, the learning device 110 includes a training image acquirer 111, a learner 112, and a parameter storage 113. Also, the moving object tracking device 320 includes a first image acquirer 321 and a tracker 122.

The medical image processing device 300 has a configuration in which the display 330 is added to the medical image processing device 100 of the first embodiment. In association with this, the medical image processing device 300 includes the moving object tracking device 320 with which the moving object tracking device 120 constituting the medical image processing device 100 of the first embodiment is replaced. Also, the moving object tracking device 320 includes the first image acquirer 321 with which the first image acquirer 121 provided in the moving object tracking device 120 constituting the medical image processing device 100 of the first embodiment is replaced. The other components provided in the medical image processing device 300 are the same as those provided in the medical image processing device 100 of the first embodiment. Accordingly, in the following description, the components of the medical image processing device 300 similar to those provided in the medical image processing device 100 of the first embodiment are denoted by the same reference signs and a detailed description of similar components will be omitted. In the following description, only components different from those of the medical image processing device 100 of the first embodiment will be described.

The moving object tracking device 320 acquires a first image, which is a fluoroscopic image PI of the patient P in which the marker to be tracked by the medical image processing device 300 is photographed, when the radiation treatment is performed, and tracks the marker photographed in the first image on the basis of a first feature of the marker learned by the learning device 110 as in the moving object tracking device 120 constituting the medical image processing device 100 of the first embodiment. Also, the moving object tracking device 320 outputs a marker position signal SM representing the position of the marker which is being tracked as in the moving object tracking device 120 constituting the medical image processing device 100 of the first embodiment. Furthermore, the moving object tracking device 320 also outputs the acquired first image and the marker position signal SM to the display 330.

Also, the moving object tracking device 320 may be configured to output information representing the range of the marker which is being tracked within the first image to the display 330 together with the marker position signal SM.

The first image acquirer 321 acquires a first image, which is a fluoroscopic image PI of the patient P captured during treatment, and outputs the acquired first image to the tracker 122 as in the first image acquirer 121 provided in the moving object tracking device 120 constituting the medical image processing device 100 of the first embodiment.

Also, the first image acquirer 321 outputs the acquired first image to the display 330.

Also, the operation of the first image acquirer 321 is similar to that of the first image acquirer 121 provided in the moving object tracking device 120 constituting the medical image processing device 100 of the first embodiment, except that the acquired first image is also output to the display 330. It can be considered that the operation of the medical image processing device 300 including the first image acquirer 321 is also similar to the operation of the moving object tracking device 120. That is, the other operation of the medical image processing device 300 including the first image acquirer 321 is similar to that of the moving object tracking device 120, except that the acquired first image is output to the display 330 in step S101 of the example of the flowchart showing the flow of the operation of the moving object tracking device 120 shown in Fig. 4. Accordingly, a detailed description of the operation of the moving object tracking device 320 will be omitted.

The display 330 is, for example, a display user interface including a display device such as a liquid crystal display (LCD). The display 330 acquires the first image and the marker position signal SM output from the moving object tracking device 320. The display 330 displays information such as a state in which the marker placed within the body of the patient P is being tracked or the position of a lesion estimated from the position of the marker which is being tracked within the acquired first image on the basis of the acquired marker position signal SM. More specifically, the display 330 displays an image for causing the position information of the marker which is being tracked represented by the marker position signal SM to be superimposed and displayed within the first image. Also, the display 330 estimates the position of the lesion photographed within the first image on the basis of the position information of the marker which is being tracked represented by the marker position signal SM and displays an image for causing the estimated position information of the lesion to be superimposed and displayed within the first image.

Also, when the moving object tracking device 320 is configured to output information representing a range of the marker which is being tracked within the first image together with the marker position signal SM, the display 330 may display an image for causing the information representing the range of the marker which is being tracked within the first image to be displayed within the first image together with the position information of the marker.

Here, a method in which the display 330 estimates the position of the lesion photographed within the first image on the basis of the marker position signal SM will be described. Here, a method in which the display 330 estimates the position of a lesion on the basis of a position $x_m$ of a rod-shaped marker M when the position of a marker which is being tracked represented by a marker position signal SM is the position $x_m$ of the rod-shaped marker M calculated by the tracker 122 provided in the moving object tracking device 120 constituting the medical image processing device 100 of the first embodiment will be described.

The display 330 calculates a position y of the lesion according to the following Eq. (5).

[Math. 5]

$$y = Ax_m + T \qquad \cdots (5)$$

In the above Eq. (5), A is a matrix of 2 rows × 2 columns. Also, in the above Eq. (5), T is a vector of 1 row×2 columns. Also, the matrix A and the vector T are obtained according to linear regression from positions of one or more rod-shaped markers M and positions of lesions obtained before the treatment and are preset in the display 330 by the radiation treatment provider (the doctor or the like) using the treatment system 3. Here, for example, the matrix A, the vector T, and the position of the rod-shaped marker M and the position of the lesion to be obtained can be obtained using a range (a region) of a lesion and a range (a region) of a marker input to (set in) a CT image captured at the time of the treatment planning. Because the CT image is a three-dimensional image, it is possible to obtain a relationship between the position of the rod-shaped marker M and the position of the lesion according to regression from the center of gravity of a range (a region) of the lesion and the center of gravity of a range (a region) of the marker projected when the DRR image is created from the CT image.

In this manner, the display 330 estimates the position of the lesion photographed within the first image output from the moving object tracking device 320 on the basis of the position information of the marker which is being tracked represented by the marker position signal SM output from the moving object tracking device 320. The display 330 displays the estimated position information of the lesion by superimposing the information within the first image.

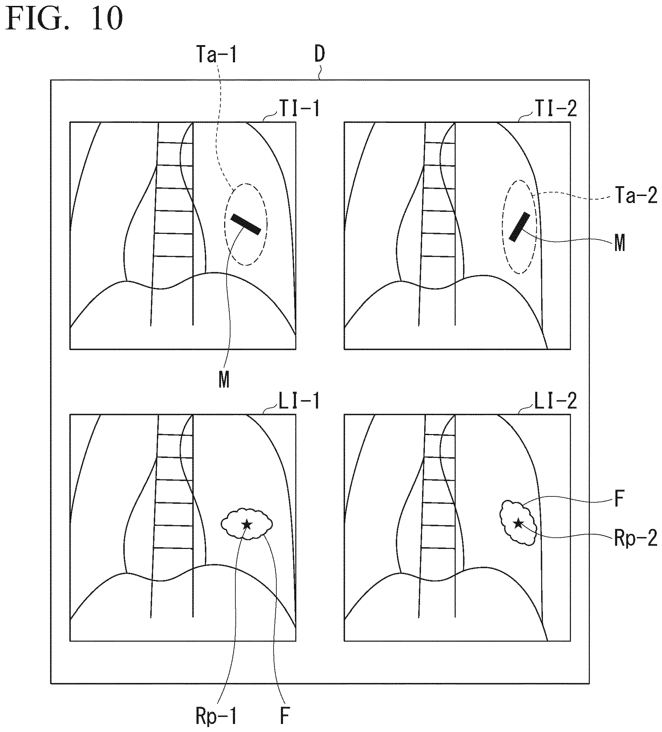

Here, an example in which the display 330 displays information such as a state in which a rod-shaped marker M placed within the body of the patient P is being tracked or the position of a lesion estimated from the position of the rod-shaped marker M which is being tracked will be described. Fig. 10 is a diagram showing information displayed by the display 330 provided in the medical image processing device 300 of the third embodiment. In Fig. 10, an example of a display screen of the display 330 on which the medical image processing device 300 displays a state in which a rod-shaped marker M placed within the body of the patient P is being tracked and position information of a lesion estimated from the position of the rod-shaped marker M which is being tracked is shown.

In Fig. 10, in the upper part of a display screen D, a state in which the medical image processing device 300 tracks the rod-shaped marker M placed within the body of the patient P is displayed in the first image captured by each of the imaging devices provided in the treatment device 10. More specifically, the rod-shaped marker M and a tracking range Ta-1 in which the rod-shaped marker M is being tracked are shown in a first image TI-1 captured by an imaging device (hereinafter referred to as an "imaging device -1") including a set of a radiation source 12-1 and a radiation detector 13-1 displayed on the left side of the upper part of the display screen D shown in Fig. 10. Also, the rod-shaped marker M and a tracking range Ta-2 in which the rod-shaped marker M is being tracked are shown in a first image TI-2 captured by an imaging device (hereinafter referred to as an "imaging device -2") including a set of a radiation source 12-2 and a radiation detector 13-2 displayed on the right side of the upper part of the display screen D shown in Fig. 10.

Also, in Fig. 10, in the lower part of the display screen D, position information of a lesion estimated from the position of the rod-shaped marker M tracked by the medical image processing device 300 and the position information of irradiation with the treatment beam B is displayed in the first image captured by each of the imaging devices provided in the treatment device 10. More specifically, the position of a lesion F estimated from the position of the rod-shaped marker M and an irradiation position Rp-1 where the lesion F is irradiated with the treatment beam B are shown in the first image LI-1 captured by the imaging device -1 displayed on the left side of the lower part of the display screen D shown in Fig. 10. Also, the position of the lesion F estimated from the position of the rod-shaped marker M and an irradiation position Rp-2 where the lesion F is irradiated with the treatment beam B are shown in a first image LI-2 captured by the imaging device -2 displayed on the right side of the lower part of the display screen D shown in Fig. 10.

A radiation treatment provider (a doctor or the like) using the treatment system 3 can monitor a state in which the rod-shaped marker M placed within the body of the patient P is being tracked, and can confirm whether or not the lesion F estimated from the position of the rod-shaped marker M which is being tracked is being treated normally, according to display content such as an example of the display screen D of the display 330 shown in Fig. 10.

As described above, in the medical image processing device 300 of the third embodiment, as in the medical image processing device 100 of the first embodiment, the learning device 110 learns a first feature common to marker images of the rod-shaped marker M placed within the body of the patient P on the basis of training images, calculates a feature extraction parameter for tracking the rod-shaped marker M on the basis of the first feature obtained through learning, and causes the parameter storage 113 to store the calculated feature extraction parameter. In the medical image processing device 300 of the third embodiment, as in the medical image processing device 100 of the first embodiment, the moving object tracking device 320 tracks the rod-shaped marker M photographed in a first image (a fluoroscopic image PI) of the patient P captured during the treatment and outputs a marker position signal SM representing the position of the rod-shaped marker M.

Thereby, in the medical image processing device 300 of the third embodiment, as in the medical image processing device 100 of the first embodiment, it is also possible to efficiently perform calculation when the rod-shaped marker M is tracked and curb deterioration of the real-time performance of tracking of the rod-shaped marker M. Also, in the medical image processing device 300 of the third embodiment, as in the medical image processing device 100 of the first embodiment, it is also possible to reduce a possibility of erroneous tracking of the rod-shaped marker M that may be caused due to the presence of a template having a high degree of similarity to the rod-shaped marker M in the conventional template matching.

Thereby, in the treatment system 3 including the medical image processing device 300 of the third embodiment, as in the treatment system 1 including the medical image processing device 100 of the first embodiment, it is also possible to safely irradiate the lesion with the treatment beam B at an appropriate timing synchronized with the expiration or inspiration of the patient P.

Also, in the medical image processing device 300 of the third embodiment, the display 330 displays information such as a state in which the rod-shaped marker M placed within the body of the patient P is being tracked or the position of a lesion estimated from the position of the rod-shaped marker M which is being tracked within the first image output from the moving object tracking device 320 on the basis of the marker position signal SM output from the moving object tracking device 320. Thereby, in the treatment system 3 including the medical image processing device 300 of the third embodiment, the radiation treatment provider (the doctor or the like) using the treatment system 3 can monitor a state in which the rod-shaped marker M placed within the body of the patient P is being tracked. Also, in the treatment system 3 including the medical image processing device 300 of the third embodiment, the radiation treatment provider (the doctor or the like) using the treatment system 3 can confirm whether or not the lesion estimated from the position of the marker which is being tracked is being treated normally.

Also, although a case in which the target marker to be tracked is the rod-shaped marker M has been described in the medical image processing device 300 of the third embodiment, the target marker to be tracked is not limited to the rod-shaped marker M as in the medical image processing device 100 of the first embodiment.

Also, in the medical image processing device 300 of the third embodiment, a case in which the display 330 acquires a first image and a marker position signal SM output from the moving object tracking device 320, estimates the position of a lesion F from the position of the rod-shaped marker M which is being tracked on the basis of the marker position signal SM, and causes the estimated position to be superimposed and displayed within the first image in the configuration of the medical image processing device 300 shown in Fig. 9 has been described. However, the component for estimating the position of the lesion F from the position of the rod-shaped marker M which is being tracked is not limited to the display 330. For example, the tracker 122 provided in the moving object tracking device 320 may be configured to estimate the position of the lesion F from the position of the rod-shaped marker M which is being tracked and output information such as the estimated position of the lesion F, the size of the lesion F, and the like to the display 330.

Also, in the medical image processing device 300 of the third embodiment, a case in which the display 330 is a component provided in the medical image processing device 300 in the configuration of the medical image processing device 300 shown in Fig. 9 has been described. However, the display 330 is not limited to the configuration in which the display 330 is a component provided in the medical image processing device 300. For example, the display 330 may be provided outside the medical image processing device 300. Also, for example, in the treatment system 3 including the medical image processing device 300 of the third embodiment, a function of the display 330, i.e., a function of displaying information or an image based on the first image and the marker position signal SM output from the moving object tracking device 320 constituting the medical image processing device 300, may be provided in the treatment device 10.

As described above, the medical image processing device 300 further includes the display 330 configured to display position information of a tracked object.

Also, as described above, in the medical image processing device 300, the display 330 may cause the first image to be displayed and cause position information of the object tracked within the first image to be superimposed and displayed within the displayed first image.

Also, as described above, in the medical image processing device 300, the display 330 may cause position information of the treatment target portion (a lesion) to be superimposed and displayed within the displayed first image.

(Fourth embodiment)

Hereinafter, a fourth embodiment will be described. Also, a configuration of a treatment system including a medical image processing device of the fourth embodiment is a configuration in which the medical image processing device 100 in the configuration of the treatment system 1 including the medical image processing device 100 of the first embodiment shown in Fig. 1 is replaced with the medical image processing device of the fourth embodiment (hereinafter referred to as a "medical image processing device 400"). In the following description, the treatment system including the medical image processing device 400 is referred to as a "treatment system 4."

Also, in the following description, the components of the treatment system 4 including the medical image processing device 400 similar to those of the treatment system 1 including the medical image processing device 100 of the first embodiment are denoted by the same reference signs and a detailed description of similar components will be omitted. In the following description, only a configuration, an operation, and a process of the medical image processing device 400, which is a component different from the medical image processing device 100 of the first embodiment, will be described.

The medical image processing device 400 tracks a marker placed within a body of a patient P to be treated in radiation treatment on the basis of fluoroscopic images PI output from a radiation detector 13-1 and a radiation detector 13-2 as in the medical image processing device 100 of the first embodiment. Thereby, in the medical image processing device 400, as in the medical image processing device 100 of the first embodiment, it is also possible to track a lesion within the body of the patient P to be treated in the radiation treatment. Also, the medical image processing device 400 performs the tracking of a marker according to template matching using a template associated with the posture of the marker placed within the body of the patient P in addition to learning based on a training image similar to that of the medical image processing device 100 of the first embodiment. In this regard, if template matching is performed using templates associated with all available postures of the marker, the real-time performance of tracking of the marker is likely to deteriorate. Thus, when the marker is tracked according to the template matching, the medical image processing device 400 limits the template used in the template matching. Thereby, the medical image processing device 400 can more robustly track the marker. The medical image processing device 400 automatically detects a timing at which the lesion will be irradiated with the treatment beam B in the radiation treatment on the basis of a result of tracking the marker placed within the body of the patient P as in the medical image processing device 100 of the first embodiment.

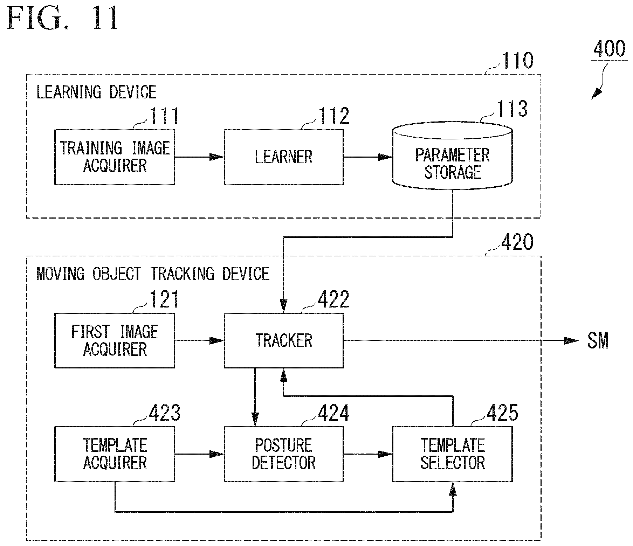

The configuration of the medical image processing device 400 constituting the treatment system 4 will be described below. Fig. 11 is a block diagram showing a schematic configuration of the medical image processing device 400 of the fourth embodiment. The medical image processing device 400 shown in Fig. 11 includes a learning device 110 and a moving object tracking device 420. Also, the learning device 110 includes a training image acquirer 111, a learner 112, and a parameter storage 113. Also, the moving object tracking device 420 includes a first image acquirer 121, a tracker 422, a template acquirer 423, a posture detector 424, and a template selector 425.

The medical image processing device 400 has a configuration in which the moving object tracking device 120 constituting the medical image processing device 100 of the first embodiment is replaced with the moving object tracking device 420. The moving object tracking device 420 has a configuration in which the template acquirer 423, the posture detector 424, and the template selector 425 are added to the moving object tracking device 120 constituting the medical image processing device 100 of the first embodiment. In association with this, the medical image processing device 400 includes the tracker 422 with which the tracker 122 provided in the moving object tracking device 120 constituting the medical image processing device 100 of the first embodiment is replaced. Also, the other components provided in the medical image processing device 400 are the same as those provided in the medical image processing device 100 of the first embodiment. Accordingly, in the following description, the components of the medical image processing device 400 similar to those provided in the medical image processing device 100 of the first embodiment are denoted by the same reference signs and a detailed description of similar components will be omitted. In the following description, only components different from those of the medical image processing device 100 of the first embodiment will be described.

The moving object tracking device 420 acquires a first image, which is a fluoroscopic image PI of the patient P in which the marker to be tracked by the medical image processing device 300 is photographed, when the radiation treatment is performed, and tracks the marker photographed in the first image on the basis of a first feature of the marker learned by the learning device 110 as in the moving object tracking device 120 constituting the medical image processing device 100 of the first embodiment. Further, the moving object tracking device 420 also tracks the marker according to template matching. Also, the moving object tracking device 420 outputs the marker position signal SM representing the position of the marker which is being tracked as in the moving object tracking device 120 constituting the medical image processing device 100 of the first embodiment.

The template acquirer 423 acquires templates of a marker image in which the marker placed within the body of the patient P is shown within the fluoroscopic image PI. The template acquirer 423 outputs the acquired templates to the posture detector 424 and the template selector 425. Here, the templates are a plurality of templates associated with all available postures of the marker placed within the body of the patient P. Each template is virtually generated while a posture parameter of the marker is being changed to various values as in a method in which the training image acquirer 111 provided in the learning device 110 constituting the medical image processing device 100 of the first embodiment acquires a positive example image. At this time, the posture parameter may limit a value to be changed to generate the template on the basis of a concept similar to that when the marker image to be learned in the medical image processing device 200 of the second embodiment is limited.

The posture detector 424 acquires a plurality of templates from the template acquirer 423. Also, the posture detector 424 acquires position information of the marker which is being tracked from the tracker 422. The posture detector 424 estimates a posture of the marker on the basis of the acquired the position information of the marker. Also, a method in which the posture detector 424 estimates the posture of the marker is similar to a method in which the object extractor 215 provided in the learning device 210 constituting the medical image processing device 200 of the second embodiment estimates the posture of the marker. The posture detector 424 outputs the estimated posture information of the marker to the template selector 425.

The template selector 425 acquires a plurality of templates from the template acquirer 423. Also, the template selector 425 also acquires posture information of a marker output from the posture detector 424. The template selector 425 selects a template associated with the posture of the marker which is being tracked from among the plurality of acquired templates on the basis of the acquired the posture information of the marker. Also, the template selected by the template selector 425 is not limited to a template associated with the posture of the marker which is being tracked, i.e., one set of templates. The marker which is being tracked moves within the body in conjunction with the respiration or heartbeat of the patient P or the like. Thus, the template selector 425 may collectively select a plurality of sets of templates in which the value of the posture parameter is slightly changed using the posture parameter of the template associated with the posture of the marker which is being tracked as a central value in consideration of the movement of the marker. In this case, because the number of templates to be selected is smaller than the number of templates associated with all available postures of the marker, it is also possible to curb unnecessary deterioration of the robustness of tracking of the marker according to template matching. The template selector 425 outputs the selected template to the tracker 422.

The tracker 422 tracks the marker photographed in the first image output from the first image acquirer 121 as in the tracker 122 provided in the moving object tracking device 120 constituting the medical image processing device 100 of the first embodiment. If no template is output from the template selector 425 when the marker is tracked, the tracker 422 tracks the marker photographed in the first image on the basis of the feature extraction parameter stored in the parameter storage 113 provided in the learning device 110 as in the tracker 122 provided in the moving object tracking device 120 constituting the medical image processing device 100 of the first embodiment. On the other hand, if the template is output from the template selector 425 when the marker is tracked, the tracker 422 tracks the marker photographed in the first image according to template matching using the output template. The tracker 122 outputs a marker position signal SM representing the position of the marker which is being tracked. The tracker 422 also outputs the position information of the marker which is being tracked to the posture detector 424.

According to this configuration, the medical image processing device 400 tracks the marker within the body of the patient P at present on the basis of the learned first feature of the marker or the selected template and outputs the marker position signal SM representing the position of the marker which is being tracked. Thereby, in the medical image processing device 200, as in the medical image processing device 100 of the first embodiment, it is also possible to automatically detect a timing at which the lesion within the body of the patient P will be irradiated with the treatment beam B. Thereby, in the treatment system 4 including the medical image processing device 400, as in the treatment system 1 including the medical image processing device 100 of the first embodiment, it is also possible to irradiate the lesion within the body of the patient P with the treatment beam B at an appropriate timing. Moreover, because a template used when the marker is tracked according to template matching is limited on the basis of a posture of the target marker to be tracked within the body of the patient P at present in the medical image processing device 400, it is possible to improve robustness as compared with marker tracking based on the conventional template matching even when the marker is tracked according to template matching.

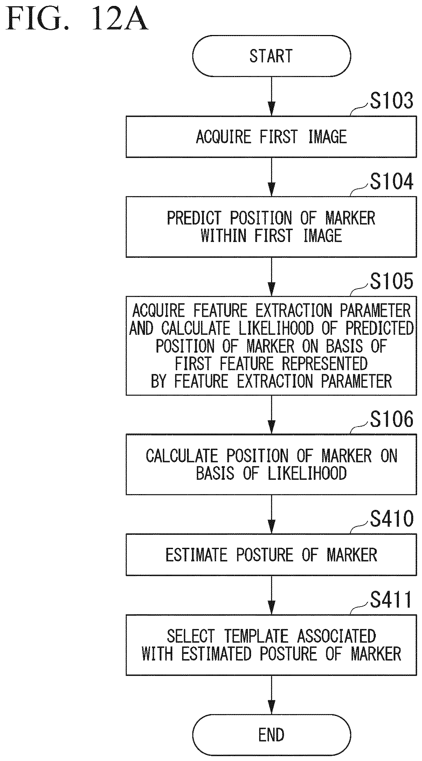

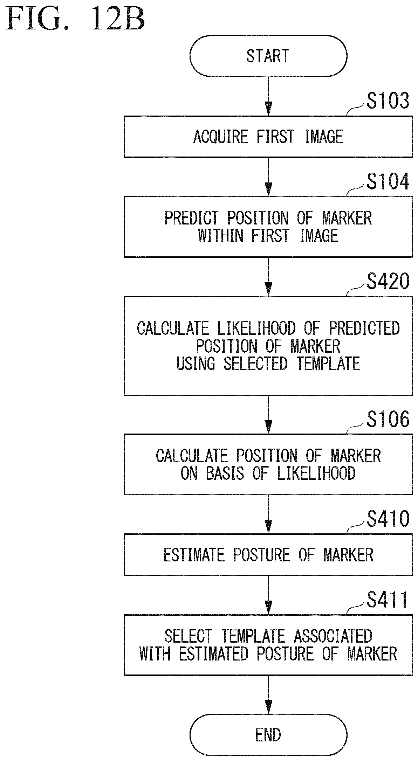

Here, the operation of the medical image processing device 400 constituting the treatment system 4 will be schematically described. Here, the operation of the moving object tracking device 420 constituting the medical image processing device 400 will be schematically described. Figs. 12A and 12B are examples of a flowchart showing the flow of the operation of the moving object tracking device 420 constituting the medical image processing device 400 of the fourth embodiment. In Fig. 12A, an example of the flow of the operation when the moving object tracking device 420 tracks a marker photographed in a first image that has been initially acquired is shown. An example of the flowchart showing the flow of the operation of the moving object tracking device 420 shown in Fig. 12A is an example of a flow of an operation in which the marker photographed in the first image that has been initially acquired is tracked on the basis of a learned feature of the marker as in the moving object tracking device 120 constituting the medical image processing device 100 of the first embodiment and a template associated with a posture of the marker is further selected on the basis of position information of the tracked marker. Also, in Fig. 12B, an example of the flow of the operation when the moving object tracking device 420 tracks the marker photographed in the first image acquired from the next time is shown. An example of the flowchart showing the flow of the operation of the moving object tracking device 420 shown in Fig. 12B is an example of the flow of the operation of tracking the marker photographed in the first image acquired from the next time according to template matching using a selected template. Also, an example of the flowchart showing the flow of the operation of the moving object tracking device 420 shown in Fig. 12 includes operations (processing) similar to those of the moving object tracking device 120 constituting the medical image processing device 100 of the first embodiment. Accordingly, in the following description, in the example of the flowchart showing the flow of the operation of the moving object tracking device 420, operations (processing) similar to those of the moving object tracking device 120 constituting the medical image processing device 100 of the first embodiment are denoted by the same step numbers and different operations (processing) will be mainly described.

First, an operation of the moving object tracking device 420 for tracking a marker photographed in a first image that has been initially acquired will be described with reference to Fig. 12A. When the moving object tracking device 120 starts an operation, the first image acquirer 121 first acquires the initial first image (step S103). Subsequently, the tracker 422 predicts a position of the marker within the initial first image output from the first image acquirer 121 (step S104). Subsequently, the tracker 422 acquires a feature extraction parameter stored in the parameter storage 113 and calculates the likelihood of the predicted marker position on the basis of a first feature represented by the acquired feature extraction parameter (step S105). Subsequently, the tracker 422 calculates the position of the marker included in the initial first image on the basis of the calculated likelihood (step S106). The tracker 422 outputs a marker position signal SM representing the position of the marker which is being tracked included in the calculated initial first image. Also, the tracker 422 outputs the position information of the marker which is being tracked to the posture detector 424. Subsequently, the posture detector 424 estimates the posture of the marker on the basis of the position information of the marker which is being tracked output from the tracker 422 (step S410). Also, the tracker 422 outputs the estimated posture information of the marker to the template selector 425. Subsequently, the template selector 425 selects a template associated with the estimated posture of the marker (which is being initially tracked) on the basis of the posture information of the marker output from the posture detector 424. The template selector 425 outputs the selected template to the tracker 422.

Next, an operation in which the moving object tracking device 420 tracks a marker photographed in a first image acquired from the next time will be described with reference to Fig. 12B. When the moving object tracking device 120 starts the operation, the first image acquirer 121 first acquires the next and subsequent first images (step S103). Subsequently, the tracker 422 predicts a position of a marker within the next and subsequent first images output from the first image acquirer 121 (step S104). Subsequently, the tracker 422 calculates the likelihood of the predicted position of the marker according to template matching using a template output from the template selector 425 (step S420). Subsequently, the tracker 422 calculates the position of the marker included in the next and subsequent first images on the basis of the calculated likelihood (step S106). The tracker 422 outputs a marker position signal SM representing the position of the marker which is being tracked included in the calculated next and subsequent first images. Also, the tracker 422 outputs the position information of the marker which is being tracked to the posture detector 424. Subsequently, the posture detector 424 estimates the posture of the marker on the basis of the position information of the marker which is being tracked output from the tracker 422 (step S410). Also, the tracker 422 outputs the estimated posture information of the marker to the template selector 425. Subsequently, the template selector 425 selects a template associated with the estimated posture of the marker (which is being tracked from the next time) on the basis of the posture information of the marker output from the posture detector 424. The template selector 425 outputs the selected template to the tracker 422.

The marker tracking having high robustness in the moving object tracking device 420 can be represented by integrating the positions of the markers tracked on the basis of the learned features of the marker shown in Fig. 12A and the positions of the markers tracked according to the template matching shown in Fig. 12B in the moving object tracking device 420.

Here, a method in which the tracker 422 provided in the moving object tracking device 420 tracks a marker photographed in a first image according to template matching using a template selected by the template selector 425 will be described. The tracker 422 calculates the likelihood of a predicted position of a marker as in the tracker 122 provided in the moving object tracking device 120 constituting the medical image processing device 100 of the first embodiment in step S420 of the example of the flowchart showing the flow of the operation of the moving object tracking device 420 shown in Fig. 12B. Also, in the following description, it is assumed that the marker placed within the body of the patient P is a marker other than a spherical marker and is a rod-shaped marker M as an example will be described.

Here, the likelihood calculated by the tracker 422 is a value representing the degree of similarity between a first image predicted and acquired by the tracker 422 from the next time, i.e., an image photographed at the position of the rod-shaped marker M within the first image during treatment, and a template. Also, normalized cross-correlation, the amount of mutual information, and the like can be used for calculating the degree of similarity. Alternatively, the degree of separation may be set as the degree of similarity.

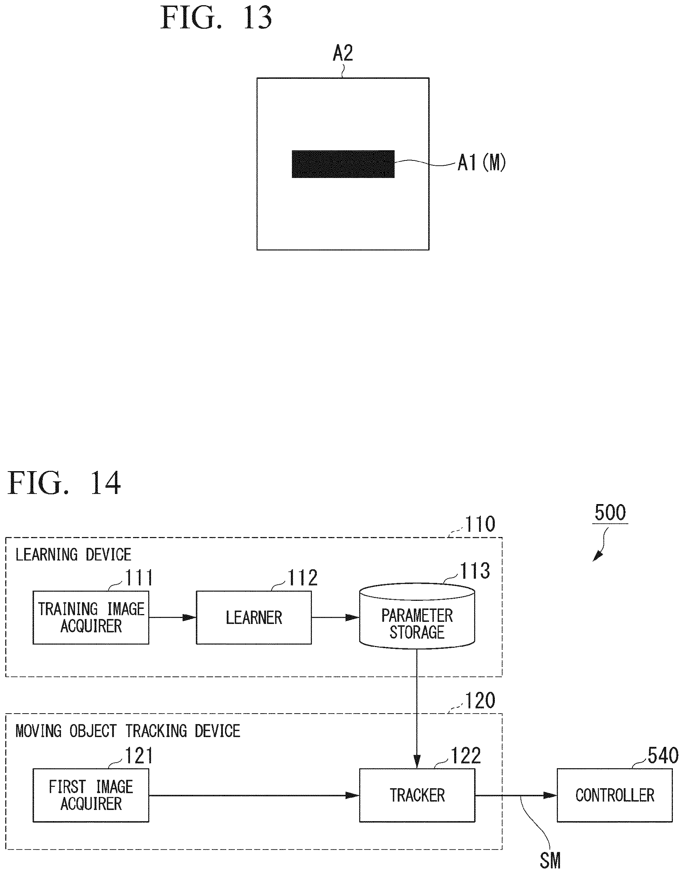

A method of calculating the degree of separation will be described below with reference to Fig. 13. Fig. 13 is a diagram showing an example of a template used for tracking a marker in the moving object tracking device 420 constituting the medical image processing device 400 of the fourth embodiment. In the example of the template shown in Fig. 13, regions are classified into a first region A1 in which the rod-shaped marker M is present and a second region A2 (i.e., a region in which the rod-shaped marker M is not present) other than the first region A1. In the calculation of the degree of separation, when a region similar to the template is present in the first image, a histogram of pixel values included in the first image is classified into each of a histogram of pixel values belonging to the first region A1 and a histogram of pixel values belonging to the second region A2. This is because, when the marker image of the rod-shaped marker M shown in the first image overlaps the first region A1, a histogram of pixel values belonging to the first region A1 has a high frequency of pixel values of dark pixels and a histogram of pixel values belonging to the second region A2 has a high frequency of pixel values of bright pixels. In the calculation of the degree of separation, Fisher's discrimination criterion is used to quantify separability of the histogram of pixel values as described above. In the calculation of the degree of separation, a ratio between an average of the variance of pixel values of pixels belonging to regions (intra-class variance) and the variance of pixel values between regions (inter-class variance) is calculated and the calculated ratio is used as the degree of separation. In this manner, the likelihood of the predicted marker position can be calculated using the template. The tracker 422 can perform robust tracking by performing tracking using a product of the calculated likelihood of a predicted marker position and the likelihood obtained by a classifier provided in the tracker 422.

As described above, in the medical image processing device 400 of the fourth embodiment, the learning device 110 also acquires a training image in the medical image processing device 400 before radiation treatment is performed, learns a first feature for detecting a marker image from the acquired training image, calculates a feature extraction parameter representing the feature of the marker on the basis of the learned first feature, and causes the parameter storage 113 to store the calculated feature extraction parameter. Also, in the medical image processing device 400 of the fourth embodiment, the template acquirer 423 provided in the moving object tracking device 420 acquires a template of a marker image of a rod-shaped marker M placed within the body of the patient P shown within a fluoroscopic image PI (a first image). The medical image processing device 400 of the fourth embodiment tracks the rod-shaped marker M photographed in the first image, which has been initially acquired, on the basis of the first feature represented by the feature extraction parameter, estimates a posture of the rod-shaped marker M on the basis of position information of the tracked rod-shaped marker M, and selects a template associated with the posture of the rod-shaped marker M. Also, the medical image processing device 400 of the fourth embodiment tracks the rod-shaped marker M photographed in a first image acquired from the next time according to template matching using the selected template, similarly estimates a posture of the rod-shaped marker M, and selects a template associated with the posture of the rod-shaped marker M. Subsequently, likewise, the medical image processing device 400 of the fourth embodiment tracks the rod-shaped marker M photographed in the acquired first image according to template matching using the selected template, similarly estimates a posture of the rod-shaped marker M, and tracks the rod-shaped marker M while selecting a template associated with the posture of the rod-shaped marker M. In the medical image processing device 400 of the fourth embodiment, as in the medical image processing device 100 of the first embodiment, the moving object tracking device 420 outputs a marker position signal SM representing a position of the tracked rod-shaped marker M.

Thereby, in the medical image processing device 400 of the fourth embodiment, as in the medical image processing device 100 of the first embodiment, it is also possible to efficiently perform calculation when the rod-shaped marker M is tracked and curb deterioration of real-time performance of tracking of the rod-shaped marker M. In the medical image processing device 400 of the fourth embodiment, the rod-shaped marker M is tracked according to template matching, but the rod-shaped marker M is tracked while the template for use in the template matching is being limited on the basis of the posture of the target rod-shaped marker M tracked within the body of the patient P at present. Thereby, in the medical image processing device 400 of the fourth embodiment, it is possible to improve robustness when the rod-shaped marker M is tracked according to template matching. Thereby, in the medical image processing device 400 of the fourth embodiment, as in the medical image processing device 100 of the first embodiment, it is also possible to reduce a possibility of erroneous tracking of the rod-shaped marker M that may be caused due to the presence of a template having a high degree of similarity to the rod-shaped marker M in the conventional template matching.

Thereby, in the treatment system 4 including the medical image processing device 300 of the fourth embodiment, as in the treatment system 1 including the medical image processing device 100 of the first embodiment, it is also possible to safely irradiate the lesion with the treatment beam B at an appropriate timing synchronized with the expiration or inspiration of the patient P.

Also, although a case in which the target marker to be tracked is the rod-shaped marker M has been described in the medical image processing device 400 of the fourth embodiment, the target marker to be tracked is not limited to the rod-shaped marker M as in the medical image processing device 100 of the first embodiment.

As described above, the medical image processing device 400 further includes the template acquirer 423 configured to acquire a template of an object image; the posture detector 424 configured to detect a posture of an object on the basis of position information of the object tracked by the tracker 422; and the template selector 425 configured to select the template associated with the posture of the object, wherein the tracker 422 tracks the object using the selected template when the template selector 425 has selected the template.

(Fifth embodiment)

Hereinafter, a fifth embodiment will be described. Also, a configuration of a treatment system including a medical image processing device of the fifth embodiment is a configuration in which the medical image processing device 100 in the configuration of the treatment system 1 including the medical image processing device 100 of the first embodiment shown in Fig. 1 is replaced with the medical image processing device of the fifth embodiment (hereinafter referred to as a "medical image processing device 500"). In the following description, the treatment system including the medical image processing device 500 is referred to as a "treatment system 5."

Also, in the following description, the components of the treatment system 5 including the medical image processing device 500 similar to those of the treatment system 1 including the medical image processing device 100 of the first embodiment are denoted by the same reference signs and a detailed description of similar components will be omitted. In the following description, only a configuration, an operation, and a process of the medical image processing device 500, which is a component different from the medical image processing device 100 of the first embodiment, will be described.

The medical image processing device 500 tracks a marker placed within a body of a patient P to be treated in radiation treatment on the basis of fluoroscopic images PI output from a radiation detector 13-1 and a radiation detector 13-2 as in the medical image processing device 100 of the first embodiment. Thereby, in the medical image processing device 500, as in the medical image processing device 100 of the first embodiment, it is also possible to track a lesion within the body of the patient P to be treated in the radiation treatment. The medical image processing device 500 automatically detects a timing at which the lesion will be irradiated with the treatment beam B in the radiation treatment on the basis of a result of tracking the marker placed within the body of the patient P as in the medical image processing device 100 of the first embodiment. The medical image processing device 500 controls the irradiation with the treatment beam B by the treatment beam irradiation gate 14 provided in the treatment device 10 or the irradiation with radiation r by the radiation source 12 on the basis of a result of tracking the marker placed within the body of the patient P.

A configuration of the medical image processing device 500 constituting the treatment system 5 will be described below. Fig. 14 is a block diagram showing a schematic configuration of the medical image processing device 500 of the fifth embodiment. The medical image processing device 500 shown in Fig. 14 includes a learning device 110, a moving object tracking device 120, and a controller 540. Also, the learning device 110 includes a training image acquirer 111, a learner 112, and a parameter storage 113. Also, the moving object tracking device 120 includes a first image acquirer 121 and a tracker 122.

The medical image processing device 500 has a configuration in which a controller 540 is added to the medical image processing device 100 of the first embodiment. The other components provided in the medical image processing device 500 are the same as those provided in the medical image processing device 100 of the first embodiment. Accordingly, in the following description, the components of the medical image processing device 500 similar to those provided in the medical image processing device 100 of the first embodiment are denoted by the same reference signs and a detailed description of similar components will be omitted. In the following description, only components different from those of the medical image processing device 100 of the first embodiment will be described.

The tracker 122 provided in the moving object tracking device 120 constituting the medical image processing device 500 outputs a marker position signal SM representing a position of the marker which is being tracked to the controller 540.

The controller 540 controls the radiation treatment in the treatment system 5 including the medical image processing device 500 on the basis of the marker position signal SM output from the tracker 122 provided in the moving object tracking device 120. More specifically, the controller 540 determines whether or not the position of a marker which is being tracked is within a predetermined range (region) where radiation treatment is performed on the basis of the position information of the tracked marker represented by the marker position signal SM output from the tracker 122 and outputs a control signal for controlling the radiation treatment in the treatment system 5. That is, the controller 540 outputs a control signal for performing control so that the radiation treatment in the treatment system 5 is performed when the position of the lesion within the body of the patient P is within the predetermined range (region) for performing the radiation treatment and the radiation treatment in the treatment system 5 is not performed when the position of the lesion within the body of the patient P is not within the predetermined range (region) for performing the radiation treatment. In the following description, the position and the predetermined range (region) where the treatment beam B is radiated to the lesion are referred to as a "gate window."

The gate window is a position or a range (a region) where the treatment beam B is radiated to be set before radiation treatment is performed as in a treatment planning stage or the like. The gate window is determined on the basis of a position of a marker designated in a CT image captured at the time of the treatment planning. In the treatment planning, a margin is preset on the basis of an error that may occur when the actual treatment is performed. Thus, a three-dimensional region where a position of a marker within the CT image is used as a center and a margin is added to a position of the center is set as the gate window. Also, the gate window may be set using a range (a region) set for the CT image as a range (a region) projected onto a DRR image created from the CT image or a first image. Also, the gate window may be set by adding a margin set in consideration of a state of the patient P immediately before the treatment is started. By setting the gate window in consideration of the above, it is possible to avoid a situation in which the patient P is irradiated with an inappropriate treatment beam B or unnecessary radiation r, i.e., so-called exposure.

Here, the treatment planning to be performed before the radiation treatment is performed will be described. In the treatment planning, energy of a treatment beam B (radiation) with which the patient P is irradiated, an irradiation direction, a shape of an irradiation range, a distribution of a dose when the treatment beam B is radiated a plurality of times, and the like are determined. More specifically, a treatment plan creator (a doctor or the like) first designates a boundary between a tumor (lesion) region and a normal tissue region, a boundary between a tumor and an important organ around the tumor, or the like with respect to a CT image captured in the treatment planning stage. In the treatment planning, a direction (a path) or an intensity of the treatment beam B to be radiated is determined on the basis of a depth from a body surface of the patient P to a position of the tumor and a size of the tumor calculated from information about the designated tumor. At this time, the position of the marker placed within the body of the patient P is also designated (input).

The designation of the boundary between the tumor region and the normal tissue region described above corresponds to designation of a position and a volume of the tumor. The volume of this tumor is referred to as a gross tumor volume (GTV), a clinical target volume (CTV), an internal target volume (ITV), a planning target volume (PTV), or the like. The GTV is a volume of the tumor capable of being visually confirmed from the image and is a volume required to be irradiated with a sufficient dose of the treatment beam B in radiation treatment. The CTV is a volume including the GTV and a latent tumor to be treated. The ITV is a volume obtained by adding a predetermined margin to the CTV in consideration of the movement of the CTV due to predicted physiological movement of the patient P and the like. The PTV is a volume obtained by adding a margin to the ITV in consideration of an error in alignment of the patient P performed when treatment is performed. The relationship of the following Expression (6) is established between these volumes.

[Math. 6]

$$GTV \in CTV \in ITV \in PTV \qquad \cdots (6)$$

Thus, in the treatment planning stage, a position and a range (a region) where the patient P is irradiated with the treatment beam B are determined by adding a margin in consideration of the error that is likely to occur in the actual treatment. For example, the error that is likely to occur in the actual treatment considered at this time is the position deviation of the patient P that is likely to occur when the patient P is positioned so that a position of a lesion or bone within the body of the patient P is aligned with a position planned at the time of the treatment planning.

In the control of radiation treatment in the controller 540, for example, radiation of the treatment beam B by the treatment beam irradiation gate 14 provided in the treatment device 10 or capturing of the fluoroscopic image PI by the radiation source 12 and the radiation detector 13 is controlled. More specifically, when it is represented that the position of the marker which is being tracked represented by the marker position signal SM output from the tracker 122, i.e., a position of a lesion within the body of the patient P, is within a gate window where the lesion is irradiated with the treatment beam B in the radiation treatment, the controller 540 outputs a control signal for performing control so that the treatment beam irradiation gate 14 is allowed to radiate the treatment beam B. Also, when it is represented that the position of the marker which is being tracked represented by the marker position signal SM is not within the gate window, but is within a range (a region) where a fluoroscopic image PI can be acquired, the controller 540 outputs a control signal for performing control so that the radiation source 12 is allowed to radiate the radiation r, the radiation r reaching the radiation detector 13 through the inside of the body of the patient P is detected, and a fluoroscopic image PI (a first image) of the inside of the body of the patient P is generated.

Also, when it is represented that the position of the marker which is being tracked represented by the marker position signal SM is in an abnormal range as in a case in which the position largely deviates from an available normal trajectory or the like, the controller 540 outputs a control signal for performing control so that the radiation of the treatment beam B by the treatment beam irradiation gate 14 is stopped. At this time, the controller 540 may output a control signal for performing control so that the irradiation with the radiation r by the radiation source 12 and the generation of the fluoroscopic image PI by the radiation detector 13, i.e., the capturing of the fluoroscopic image PI (the first image), are stopped. By performing control in this manner, it is possible to avoid inappropriate radiation of the treatment beam B or unnecessary radiation r to the patient P.

Also, as a cause of an unexpected situation in which the position of the marker which is being tracked moves to an abnormal range, coughing or sneezing of the patient P, occurrence of an apnea syndrome while the patient P is sleeping, and the like are considered. It is considered that an unexpected situation due to such a cause will be eliminated without requiring a long time period, i.e., coughing or sneezing of the patient P or the like will subside and a stable state in which radiation treatment can be resumed will be achieved. Thus, the controller 540 may perform control so that an interval for capturing the fluoroscopic image PI (the first image) is lengthened by a predetermined time period in a state in which a process of capturing the fluoroscopic image PI (the first image) is not immediately stopped when it is represented that the position of the marker which is being tracked is in an abnormal range and a process of capturing the fluoroscopic image PI (the first image) is stopped when it is represented that the position of the marker which is being tracked is in an abnormal range thereafter.

According to this configuration and operation, the medical image processing device 500 tracks the marker within the body of the patient P at present on the basis of the learned first feature of the marker or the selected template and outputs the marker position signal SM representing the position of the marker which is being tracked as in the treatment system 1 including the medical image processing device 100 of the first embodiment. The medical image processing device 500 automatically detects a timing at which the lesion within the body of the patient P will be irradiated with the treatment beam B or a timing at which a fluoroscopic image PI (a first image) of the inside of the body of the patient P will be captured during treatment and an unexpected situation on the basis of the position of the marker which is being tracked represented by the marker position signal SM. In the medical image processing device 500, the controller 540 controls the irradiation with the treatment beam B by the treatment beam irradiation gate 14 provided in the treatment device 10 or the irradiation with the radiation r by the radiation source 12 on the basis of a detection result. Thereby, in the treatment system 5 including the medical image processing device 500, as in the treatment system 1 including the medical image processing device 100 of the first embodiment, it is also possible to irradiate the lesion within the body of the patient P with the treatment beam B at an appropriate timing. Also, in the medical image processing device 500, the controller 540 performs control so that the irradiation with the treatment beam B and the radiation r for the patient P is stopped when an unexpected situation of the patient P has been detected. Thereby, the treatment system 5 including the medical image processing device 500 can more stably perform radiation treatment.

As described above, in the medical image processing device 500 of the fifth embodiment, as in the medical image processing device 100 of the first embodiment, the learning device 110 learns a first feature common to marker images of a maker placed within the body of the patient P on the basis of training images, calculates a feature extraction parameter for tracking the marker on the basis of the first feature obtained through learning, and causes the parameter storage 113 to store the feature extraction parameter. In the medical image processing device 500 of the fifth embodiment, as in the medical image processing device 100 of the first embodiment, the moving object tracking device 120 tracks a marker photographed in the first image (the fluoroscopic image PI) of the patient P captured during the treatment and outputs a marker position signal SM representing a position of the marker.

Thereby, in the medical image processing device 500 of the fifth embodiment, as in the medical image processing device 100 of the first embodiment, it is also possible to efficiently perform calculation when the marker is tracked and curb deterioration of real-time performance of tracking of the marker. Also, in the medical image processing device 500 of the fifth embodiment, as in the medical image processing device 100 of the first embodiment, it is possible to reduce a possibility of erroneous tracking of the marker that may be caused due to the presence of a template having a high degree of similarity to the marker in the conventional template matching.

Thereby, in the treatment system 5 including the medical image processing device 500 of the fifth embodiment, as in the treatment system 1 including the medical image processing device 100 of the first embodiment, it is also possible to safely irradiate the lesion with the treatment beam B at an appropriate timing synchronized with the expiration or inspiration of the patient P.

Also, in the medical image processing device 500 of the fifth embodiment, the controller 540 controls the irradiation or stopping of the treatment beam B and the radiation r in the treatment device 10 on the basis of the marker position signal SM. Thereby, the treatment system 5 including the medical image processing device 500 of the fifth embodiment can safely perform radiation treatment in accordance with a situation in which the position of the marker placed within the body of the patient P during the treatment is tracked. That is, the treatment system 5 including the medical image processing device 500 of the fifth embodiment can reduce a possibility of inappropriate irradiation with the treatment beam B or unnecessary irradiation with radiation r for normal tissue of the patient P.

Also, in the medical image processing device 500 of the fifth embodiment, a case in which the controller 540 is a component provided in the medical image processing device 500 in the configuration of the medical image processing device 500 shown in Fig. 14 has been described. However, the controller 540 is not limited to the configuration in which the controller 540 is a component provided in the medical image processing device 500. For example, in the treatment system 5 including the medical image processing device 500 of the fifth embodiment, a configuration in which the function of the controller 540, i.e., a function of controlling the radiation treatment (controlling the irradiation with the treatment beam B or the radiation r) in the treatment system 5 on the basis of the marker position signal SM output from the tracker 122 provided in the moving object tracking device 120 constituting the medical image processing device 500, is provided in the treatment device 10 may be adopted.

As described above, the medical image processing device 500 further includes the controller 540 configured to control the treatment of a target portion (a lesion) to be treated on the basis of position information of an object tracked by the tracker 122.

Also, as described above, the controller 540 may control the photography of the object on the basis of the position information of the object tracked by the tracker 122.

As described above, the medical image processing device of each embodiment learns a feature common to markers using simulation images obtained by performing simulation in all available directions of the marker when the marker is placed within the body of the patient before treatment as training images, calculates a feature extraction parameter representing a feature of a direction of the marker on the basis of the feature obtained through the learning, and causes the parameter storage to store the feature extraction parameter. In the medical image processing device of each embodiment, the marker photographed in the fluoroscopic image PI of the patient captured during the treatment is tracked on the basis of the feature of the direction of the marker represented by the feature extraction parameter and a marker position signal SM representing the position of the marker placed within the body of the patient is output. Thereby, the medical image processing device of each embodiment can efficiently perform the calculation when the marker is tracked, curb the deterioration of real-time performance of the tracking of the marker, and reduce a possibility of erroneous tracking of the marker. Thereby, the treatment system including the medical image processing device of each embodiment can automatically detect an appropriate timing at which a moving lesion will be irradiated with the treatment beam in conjunction with the respiration or heartbeat of the patient or the like on the basis of the position of the marker which is being tracked. The treatment system including the medical image processing device of each embodiment can safely perform radiation treatment in which the lesion is irradiated with the treatment beam at an appropriate timing synchronized with the expiration or inspiration of the patient.

Also, a configuration in which characteristic components in the embodiments are added to the medical image processing device 100 of the first embodiment has been described in the second to fifth embodiments. However, the characteristic components in each embodiment are not limited to the configuration exclusively provided in the medical image processing device. That is, characteristic components of the embodiments may be simultaneously provided in the medical image processing device. For example, a medical image processing device including both the display 330 provided in the medical image processing device 300 of the third embodiment and the controller 540 provided in the medical image processing device 500 of the fifth embodiment may be provided. In this case, the other components provided in the medical image processing device implement the functions associated with the components by making an appropriate change.

Also, a configuration in which the medical image processing device and the treatment device 10 are separate devices has been described in each embodiment. However, the medical image processing device and the treatment device 10 are not limited to the configuration of separate devices and the medical image processing device and the treatment device 10 may be integrated.

A medical image processing program for use in the treatment system described in the above-described embodiment is a medical image processing program for causing a computer to function as a medical image processing device including: a first image acquirer configured to acquire a fluoroscopic image PI of a patient as a first image; and a tracker configured to track an object photographed in the first image on the basis of a first feature common to object images that are a plurality of images of the object obtained by observing the object placed within a body of the patient in a plurality of directions.

According to at least one embodiment described above, there are provided a first image acquirer (121) configured to acquire a fluoroscopic image PI of a patient P as a first image; and a tracker (122) configured to track an object (a marker) photographed in the first image on the basis of a first feature common to object images (marker images) that are a plurality of images of the object obtained by observing the object placed within a body of the patient P in a plurality of directions, so that it is possible to automatically track the marker placed within the body of the patient from the fluoroscopic image PI of the patient while radiation is radiated in radiation treatment.

Also, the various functions described above according to the treatment system of each embodiment described above may be performed by recording a program for implementing the functions of the components, which constitute the medical image processing device, such as, for example, the learning device 110 including the training image acquirer 111, the learner 112, and the parameter storage 113 or the moving object tracking device 120 including the first image acquirer 121, and the tracker 122 shown in Fig. 2, on a computer-readable recording medium and causing a computer system to read and execute the program recorded on the recording medium. Also, the "computer system" used here may include an operating system (OS) and hardware such as peripheral devices. Also, the "computer system" is assumed to include a homepage providing environment (or displaying environment) when a World Wide Web (WWW) system is used. Also, the "computer-readable recording medium" refers to a storage device such as a flexible disc, a magneto-optical disc, a read-only memory (ROM), a writable non-volatile memory such as a flash memory, a portable medium such as a compact disc-ROM (CD-ROM), and a hard disk embedded in the computer system.

Furthermore, the "computer-readable recording medium" is assumed to include a medium that holds a program for a constant period of time, such as a volatile memory (for example, a dynamic random access memory (DRAM)) inside a computer system serving as a server or a client when the program is transmitted via a network such as the Internet or a communication circuit such as a telephone circuit. Also, the above-described program may be transmitted from a computer system storing the program in a storage device or the like to another computer system via a transmission medium or by transmission waves in a transmission medium. Here, the "transmission medium" for transmitting the program refers to a medium having a function of transmitting information as in a network (a communication network) such as the Internet or a communication circuit (a communication line) such as a telephone circuit. Also, the above-described program may be a program for implementing some of the above-described functions. Further, the above-described program may be a program capable of implementing the above-described function in combination with a program already recorded on the computer system, i.e., a so-called differential file (differential program).

While several embodiments of the present invention have been described, these embodiments have been presented by way of example only, and are not intended to limit the scope of the inventions.

[Reference Signs List]

1, 2, 3, 4, 5 Treatment system
10 Treatment device
11 Treatment table
12, 12-1, 12-2 Radiation source
13, 13-1, 13-2 Radiation detector
14 Treatment beam irradiation gate
100, 200, 300, 400, 500 Medical image processing device
110, 210 Learning device
111, 211 Training image acquirer
112 Learner
113 Parameter storage
120, 320, 420 Moving object tracking device
121,321 First image acquirer
122,422 Tracker
214 Second image acquirer
215 Object extractor
330 Display

423 Template acquirer
424 Posture detector
425 Template selector
540 Controller

**Claims**

1. A medical image processing device (100, 200, 300, 400, 500) comprising:

   a first image acquirer (121, 321) configured to acquire a fluoroscopic image of a patient as a first image;
   a tracker (122, 422) configured to track an object photographed in the first image on the basis of a first feature common to object images that are a plurality of images of the object obtained by observing the object placed within a body of the patient in a plurality of directions;
   a training image acquirer (111, 211) configured to acquire a plurality of training images associated with the plurality of object images; and
   a learner (112) configured to learn the first feature common to the object images included in the plurality of training images,
   wherein the training image acquirer (111, 211) is configured to acquire the training image based on a simulation image obtained by simulating the object image in a case where the object is photographed in the first image on the basis of geometry information of an imaging device that is configured to capture the first image and a three-dimensional shape of the object.

2. The medical image processing device (100, 200, 300, 400, 500) according to claim 1,
   wherein the training image is an image in which the simulation image and the first image are combined.

3. The medical image processing device (100, 200, 300, 400, 500) according to claim 1,
   wherein the training image is an image in which the simulation image and a clinical image obtained by photographing a range, which is the same as that of the first image, are combined.

4. The medical image processing device (100, 200, 300, 400, 500) according to claim 1,
   wherein the training image includes a positive example image of a range that is predetermined to locate a center of gravity of the object image at a center thereof and a negative example image in which the center of gravity of the object image is different from that in a state of the positive example image and which has the same range as the positive example image.

5. The medical image processing device (100, 200, 300, 400, 500) according to claim 1, further comprising:

   a second image acquirer (214) configured to acquire the fluoroscopic image captured at a time different from that of the first image as a second image; and
   an object extractor (215) configured to extract the object image photographed in the second image,
   wherein the training image acquirer (111, 211) is configured to acquire the training image associated with a posture of the object photographed in the second image.

6. The medical image processing device (100, 200, 300, 400, 500) according to claim 5,
   wherein the training image is an image in which the object image extracted from the second image and a clinical image in which a range, which is the same as that of the second image, is photographed are combined.

7. The medical image processing device (100, 200, 300, 400, 500) according to claim 1, further comprising:

   a template acquirer (423) configured to acquire a template of the object image;
   a posture detector (424) configured to detect a posture of the object on the basis of position information of the object tracked by the tracker (122, 422); and
   a template selector (425) configured to select the template associated with the posture of the object,
   wherein the tracker (122, 422) is configured to track the object using the selected template in a case where the template selector (425) has selected the template.

8. The medical image processing device (100, 200, 300, 400, 500) according to claim 1,

wherein the first image is displayed and position information of the object tracked within the first image is superimposed and displayed within the displayed first image.

**9.** The medical image processing device (100, 200, 300, 400, 500) according to claim 8,
wherein position information of a treatment target portion is superimposed and displayed within the displayed first image.

**10.** The medical image processing device (100, 200, 300, 400, 500) according to claim 1, further comprising:
a controller (540) configured to control treatment on a treatment target portion on the basis of position information of the object tracked by the tracker (122, 422).

**11.** The medical image processing device (100, 200, 300, 400, 500) according to claim 10,
wherein the controller (540) is configured to control photography of the object on the basis of the position information of the object tracked by the tracker (122, 422).

**12.** The medical image processing device (100, 200, 300, 400, 500) according to claim 1,
wherein the object is a marker placed within the body of the patient.

**13.** The medical image processing device (100, 200, 300, 400, 500) according to claim 12,
wherein the marker has a shape other than a spherical shape.

**14.** A treatment system (1, 2, 3, 4, 5) comprising:

the medical image processing device (100, 200, 300, 400, 500) according to claim 1;
a treatment device (10) comprising an irradiator configured to irradiate a treatment target portion with a treatment beam and an imaging device configured to photograph the object; and
a controller (540) configured to control treatment on the treatment target portion on the basis of position information of the tracked object.

**15.** The treatment system (1, 2, 3, 4, 5) according to claim 14, further comprising:
a display (330) configured to display the position information of the tracked object.

**16.** A medical image processing program for causing a computer to function as a medical image processing device (100, 200, 300, 400, 500) comprising:

a first image acquirer (121, 321) configured to acquire a fluoroscopic image of a patient as a first image;
a tracker (122, 422) configured to track an object photographed in the first image on the basis of a first feature common to object images that are a plurality of images of the object obtained by observing the object placed within a body of the patient in a plurality of directions;
a training image acquirer (111, 211) configured to acquire a plurality of training images associated with the plurality of object images; and
a learner (112) configured to learn the first feature common to the object images included in the plurality of training images,
wherein the training image acquirer (111, 211) is configured to acquire the training image based on a simulation image obtained by simulating the object image in a case where the object is photographed in the first image on the basis of geometry information of an imaging device that is configured to capture the first image and a three-dimensional shape of the object.

**Patentansprüche**

**1.** Eine medizinische Bildverarbeitungsvorrichtung (100, 200, 300, 400, 500), aufweisend:

eine erste Bildakquirierungseinrichtung (121, 321), die konfiguriert ist, um ein Durchleuchtungsbild von einem Patienten als ein erstes Bild zu akquirieren;
eine Verfolgungseinrichtung (122, 422), die konfiguriert ist, um ein in dem ersten Bild fotografiertes Objekt auf der Basis eines ersten Merkmals zu verfolgen, das Objektbildern gemeinsam ist, die eine Vielzahl von Bilder des Objekts darstellen, das durch Beobachten des innerhalb eines Körpers des Patienten angeordneten Objekts in einer Vielzahl von Richtungen erhalten wird;
eine Trainingsbild-Akquirierungseinrichtung (111, 211), die konfiguriert ist, um eine Vielzahl von Trainingsbildern, die mit der Vielzahl von Objektbildern verknüpft sind, zu akquirieren; und
eine Lerneinrichtung (112), die konfiguriert ist, um das erste Merkmal zu lernen, das den Objektbildern gemeinsam ist, die in der Vielzahl von Trainingsbildern enthalten sind,
wobei die Trainingsbild-Akquirierungseinrichtung (111, 211) konfiguriert ist, um das Trainingsbild basierend auf einem Simulationsbild zu akquirieren, das durch Simulieren des Objektbilds in einem Fall erhalten wird, in dem das Objekt in dem ersten Bild fotografiert wird, auf der Basis von Geometrieinformationen einer Abbildungsvorrichtung, die konfiguriert ist, um das erste Bild und eine dreidimensionale Form des Objekts zu erfassen.

2. Die medizinische Bildverarbeitungsvorrichtung (100, 200, 300, 400, 500) nach Anspruch 1,
wobei das Trainingsbild ein Bild ist, in dem das Simulationsbild und das erste Bild kombiniert sind.

3. Die medizinische Bildverarbeitungsvorrichtung (100, 200, 300, 400, 500) nach Anspruch 1,
wobei das Trainingsbild ein Bild ist, in dem das Simulationsbild und ein klinisches Bild, das durch Fotografieren eines Bereichs erhalten wird, der dem des ersten Bilds entspricht, kombiniert sind.

4. Die medizinische Bildverarbeitungsvorrichtung (100, 200, 300, 400, 500) nach Anspruch 1,
wobei das Trainingsbild ein positives Beispielsbild eines Bereichs umfasst, der vorbestimmt ist, um ein Schwerkraftzentrum des Objektbilds in einem Mittelpunkt davon zu lokalisieren, und ein negatives Beispielsbild, in dem das Schwerkraftzentrum des Objektbilds von dem in einem Zustand des positiven Beispielsbilds verschieden ist, und das denselben Bereich für das positive Beispielsbild aufweist.

5. Die medizinische Bildverarbeitungsvorrichtung (100, 200, 300, 400, 500) nach Anspruch 1, ferner aufweisend:

eine zweite Bildakquirierungseinrichtung (214), die konfiguriert ist, um das Durchleuchtungsbild als ein zweites Bild zu akquirieren, das zu einem Zeitpunkt erfasst wird, der von dem des ersten Bilds verschieden ist; und
eine Objektextraktionseinrichtung (215), die konfiguriert ist, um das in dem zweiten Bild fotografierte Objektbild zu extrahieren,
wobei die Trainingsbild-Akquirierungseinrichtung (111, 211) konfiguriert ist, um das Trainingsbild zu akquirieren, das mit einer Haltung des in dem zweiten Bild fotografierten Objekts verknüpft ist.

6. Die medizinische Bildverarbeitungsvorrichtung (100, 200, 300, 400, 500) nach Anspruch 5,
wobei das Trainingsbild ein Bild ist, in dem das aus dem zweiten Bild extrahierte Objektbild und ein klinisches Bild, in dem ein Bereich fotografiert ist, der dem des zweiten Bilds entspricht, kombiniert sind.

7. Die medizinische Bildverarbeitungsvorrichtung (100, 200, 300, 400, 500) nach Anspruch 1, ferner aufweisend:

eine Vorlagenakquirierungseinheit (423), die konfiguriert ist, um eine Vorlage des Objektbilds zu akquirieren;
eine Haltungserfassungseinrichtung (424), die konfiguriert ist, um eine Haltung des Objekts auf der Basis von Positionsinformationen des durch die Verfolgungseinrichtung (122, 422) verfolgten Objekts zu erfassen; und
eine Vorlagenauswahleinrichtung (425), die konfiguriert ist, um die mit der Haltung des Objekts verknüpfte Vorlage auszuwählen,
wobei die Verfolgungseinrichtung (122, 422) konfiguriert ist, um das Objekt unter Verwendung der ausgewählten Vorlage in einem Fall zu verfolgen, in dem die Vorlagenauswahleinrichtung (425) die Vorlage ausgewählt hat.

8. Die medizinische Bildverarbeitungsvorrichtung (100, 200, 300, 400, 500) nach Anspruch 1,
wobei das erste Bild angezeigt wird und Positionsinformationen des innerhalb des ersten Bilds verfolgten Objekts innerhalb des angezeigten ersten Bilds überlagert und angezeigt werden.

9. Die medizinische Bildverarbeitungsvorrichtung (100, 200, 300, 400, 500) nach Anspruch 8,
wobei Positionsinformationen eines Behandlungszielabschnitts innerhalb des angezeigten ersten Bilds überlagert und angezeigt werden.

10. Die medizinische Bildverarbeitungsvorrichtung (100, 200, 300, 400, 500) nach Anspruch 1, ferner aufweisend:

eine Steuerung (540), die konfiguriert ist, um eine Behandlung an einem Behandlungszielabschnitt auf der Basis von Positionsinformationen des durch die Verfolgungseinrichtung (122, 422) verfolgten Objekts zu steuern.

**11.** Die medizinische Bildverarbeitungsvorrichtung (100, 200, 300, 400, 500) nach Anspruch 10, wobei die Steuerung (540) konfiguriert ist, um eine Fotografie des Objekts auf der Basis der Positionsinformationen des durch die Verfolgungseinrichtung (122, 422) verfolgten Objekts zu steuern.

**12.** Die medizinische Bildverarbeitungsvorrichtung (100, 200, 300, 400, 500) nach Anspruch 1, wobei das Objekt ein Marker ist, der innerhalb des Körpers des Patienten angeordnet ist.

**13.** Die medizinische Bildverarbeitungsvorrichtung (100, 200, 300, 400, 500) nach Anspruch 12, wobei der Marker eine Form aufweist, die von einer Sphärenform verschieden ist.

**14.** Ein Behandlungssystem (1, 2, 3, 4, 5), aufweisend:

die medizinische Bildverarbeitungsvorrichtung (100, 200, 300, 400, 500) nach Anspruch 1;
eine Behandlungsvorrichtung (10) mit einer Bestrahlungseinrichtung, die konfiguriert ist, um einen Behandlungszielabschnitt mit einem Behandlungsstrahl zu bestrahlen, und einer Abbildungsvorrichtung, die konfiguriert ist, um das Objekt zu fotografieren; und
eine Steuerung (540), die konfiguriert ist, um eine Behandlung an dem Behandlungszielabschnitt auf der Basis von Positionsinformationen des verfolgten Objekts zu steuern.

**15.** Das Behandlungssystem (1, 2, 3, 4, 5) nach Anspruch 14, ferner aufweisend:
eine Anzeige (330), die konfiguriert ist, um die Positionsinformationen des verfolgten Objekts anzuzeigen.

**16.** Ein medizinisches Bildverarbeitungsprogramm, um einen Computer zu veranlassen, als eine medizinische Bildverarbeitungsvorrichtung (100, 200, 300, 400, 500) zu fungieren, aufweisend:

eine erste Bildakquirierungseinrichtung (121, 321), die konfiguriert ist, um ein Durchleuchtungsbild von einem Patienten als ein erstes Bild zu akquirieren;
eine Verfolgungseinrichtung (122, 422), die konfiguriert ist, um ein in dem ersten Bild fotografiertes Objekt auf der Basis eines ersten Merkmals zu verfolgen, das Objektbildern gemeinsam ist, die eine Vielzahl von Bilder des Objekts darstellen, das durch Beobachten des innerhalb eines Körpers des Patienten angeordneten Objekts in einer Vielzahl von Richtungen erhalten wird;
eine Trainingsbild-Akquirierungseinrichtung (111, 211), die konfiguriert ist, um eine Vielzahl von Trainingsbildern, die mit der Vielzahl von Objektbildern verknüpft sind, zu akquirieren; und
eine Lerneinrichtung (112), die konfiguriert ist, um das erste Merkmal zu lernen, das den Objektbildern gemeinsam ist, die in der Vielzahl von Trainingsbildern enthalten sind,
wobei die Trainingsbild-Akquirierungseinrichtung (111, 211) konfiguriert ist, um das Trainingsbild basierend auf einem Simulationsbild zu akquirieren, das durch Simulieren des Objektbilds in einem Fall erhalten wird, in dem das Objekt in dem ersten Bild fotografiert wird, auf der Basis von Geometrieinformationen einer Abbildungsvorrichtung, die konfiguriert ist, um das erste Bild und eine dreidimensionale Form des Objekts zu erfassen.

**Revendications**

**1.** Dispositif de traitement d'image médicale (100, 200, 300, 400, 500) comprenant :

un premier dispositif d'acquisition d'image (121, 321) configuré pour acquérir une image fluoroscopique d'un patient en tant qu'une première image ;
un dispositif de suivi (122, 422) configuré pour suivre un objet photographié dans la première image sur la base d'une première caractéristique commune à des images d'objet qui sont une pluralité d'images de l'objet obtenues par l'observation de l'objet placé à l'intérieur d'un corps du patient dans une pluralité de directions ;
un dispositif d'acquisition d'images d'entraînement (111, 211) configuré pour acquérir une pluralité d'images d'entraînement associées à la pluralité d'images d'objet ; et
un dispositif d'apprentissage (112) configuré pour apprendre la première caractéristique commune aux images d'objet comprises dans la pluralité d'images d'entraînement,
dans lequel le dispositif d'acquisition d'images d'entraînement (111, 211) est configuré pour acquérir l'image d'entraînement sur la base d'une image de simulation obtenue par la simulation de l'image d'objet dans un cas où l'objet est photographié dans la première image sur la base d'informations de géométrie d'un dispositif d'imagerie qui est configuré pour capturer la première image et une forme tridimensionnelle de l'objet.

**2.** Dispositif de traitement d'image médicale (100, 200, 300, 400, 500) selon la revendication 1,
dans lequel l'image d'entraînement est une image dans laquelle l'image de simulation et la première image sont combinées.

**3.** Dispositif de traitement d'image médicale (100, 200, 300, 400, 500) selon la revendication 1,
dans lequel l'image d'entraînement est une image dans laquelle l'image de simulation et une image clinique obtenue par la photographie d'une plage, qui est la même que celle de la première image, sont combinées.

**4.** Dispositif de traitement d'image médicale (100, 200, 300, 400, 500) selon la revendication 1,
dans lequel l'image d'entraînement comporte une image d'exemple positif d'une plage qui est prédéterminée pour localiser un centre de gravité de l'image d'objet à un centre de celle-ci et une image d'exemple négatif dans laquelle le centre de gravité de l'image d'objet est différent de celui dans un état de l'image d'exemple positif et ayant la même plage que l'image d'exemple positif.

**5.** Dispositif de traitement d'image médicale (100, 200, 300, 400, 500) selon la revendication 1, comprenant en outre :

un deuxième dispositif d'acquisition d'images (214) configuré pour acquérir l'image fluoroscopique capturée à un temps différent de celui de la première image en tant qu'une deuxième image ; et
un dispositif d'extraction d'objet (215) configuré pour extraire l'image d'objet photographiée dans la deuxième image,
dans lequel le dispositif d'acquisition d'image d'entraînement (111, 211) est configuré pour acquérir l'image d'entraînement associée à une posture de l'objet photographié dans la deuxième image

**6.** Dispositif de traitement d'image médicale (100, 200, 300, 400, 500) selon la revendication 5,
dans lequel l'image d'entraînement est une image dans laquelle l'image d'objet extraite de la deuxième image et une image clinique dans laquelle une plage, qui est la même que celle de la deuxième image, est photographiée sont combinées.

**7.** Dispositif de traitement d'image médicale (100, 200, 300, 400, 500) selon la revendication 1, comprenant en outre :

un dispositif d'acquisition de modèle (423) configuré pour acquérir un modèle de l'image d'objet ;
un dispositif de détection de posture (424) configuré pour détecter une posture de l'objet sur la base d'informations de position de l'objet suivi par le dispositif de suivi (122, 422) ; et
un dispositif de sélection de modèle (425) configuré pour sélectionner le modèle associé à la posture de l'objet ;
dans lequel le dispositif de suivi (122, 422) est configuré pour suivre l'objet en utilisant le modèle sélectionné dans un cas où le dispositif de sélection de modèle (425) a sélectionné le modèle.

**8.** Dispositif de traitement d'image médicale (100, 200, 300, 400, 500) selon la revendication 1,
dans lequel la première image est affichée et des informations de position de l'objet suivi à l'intérieur de la première image sont superposées et affichées à l'intérieur de la première image affichée.

**9.** Dispositif de traitement d'image médicale (100, 200, 300, 400, 500) selon la revendication 8,
dans lequel des informations de position d'une partie cible de traitement sont superposées et affichées à l'intérieur de la première image affichée.

**10.** Dispositif de traitement d'image médicale (100, 200, 300, 400, 500) selon la revendication 1, comprenant en outre :
un dispositif de commande (540) configuré pour commander un traitement sur une partie cible de traitement sur la base d'informations de position de l'objet suivi par le dispositif de suivi (122, 422).

**11.** Dispositif de traitement d'image médicale (100, 200, 300, 400, 500) selon la revendication 10,
dans lequel le dispositif de commande (540) est configuré pour commander une photographie de l'objet sur la base des informations de position de l'objet suivi par le dispositif de suivi (122, 422).

**12.** Dispositif de traitement d'image médicale (100, 200, 300, 400, 500) selon la revendication 1,
dans lequel l'objet est un marqueur placé à l'intérieur du corps du patient.

**13.** Dispositif de traitement d'image médicale (100, 200, 300, 400, 500) selon la revendication 12,
dans lequel le marqueur présente une forme autre qu'une forme sphérique.

**14.** Système de traitement (1, 2, 3, 4, 5) comprenant :

le dispositif de traitement d'image médicale (100, 200, 300, 400, 500) selon la revendication 1 ;
un dispositif de traitement (10) comprenant un dispositif d'irradiation configuré pour irradier une partie cible de traitement avec un faisceau de traitement et un dispositif d'imagerie configuré pour photographier l'objet ; et
un dispositif de commande (540) configuré pour commander un traitement de la partie cible de traitement sur la base d'informations de position de l'objet suivi.

**15.** Système de traitement (1, 2, 3, 4, 5) selon la revendication 14, comprenant en outre :
un affichage (330) configuré pour afficher les informations de position de l'objet suivi.

**16.** Programme de traitement d'image médicale pour amener un ordinateur à fonctionner en tant que dispositif de traitement d'image médicale (100, 200, 300, 400, 500) comprenant :

un premier dispositif d'acquisition d'image (121, 321) configuré pour acquérir une image fluoroscopique d'un patient en tant qu'une première image ;
un dispositif de suivi (122, 422) configuré pour suivre un objet photographié dans la première image sur la base d'une première caractéristique commune à des images d'objet qui sont une pluralité d'images de l'objet obtenues par l'observation de l'objet placé à l'intérieur d'un corps du patient dans une pluralité de directions ;
un dispositif d'acquisition d'images d'entraînement (111, 211) configuré pour acquérir une pluralité d'images d'entraînement associées à la pluralité d'images d'objet ; et
un dispositif d'apprentissage (112) configuré pour apprendre la première caractéristique commune aux images d'objet comprises dans la pluralité d'images d'entraînement,
dans lequel le dispositif d'acquisition d'images d'entraînement (111, 211) est configuré pour acquérir l'image d'entraînement sur la base d'une image de simulation obtenue par la simulation de l'image d'objet dans un cas où l'objet est photographié dans la première image sur la base d'informations de géométrie d'un dispositif d'imagerie qui est configuré pour capturer la première image et une forme tridimensionnelle de l'objet.

## FIG. 1

MEDICAL IMAGE PROCESSING DEVICE 100

TREATMENT DEVICE

## FIG. 2

# FIG. 3

```
┌─────────────────┐
│      START      │
└─────────────────┘
         │
         ▼                        S100
┌─────────────────────────────────┐
│     ACQUIRE TRAINING IMAGES     │
└─────────────────────────────────┘
         │
         ▼                        S101
┌─────────────────────────────────┐
│  LEARN FIRST FEATURE COMMON TO  │
│    MARKER IMAGES INCLUDED IN    │
│        TRAINING IMAGES          │
└─────────────────────────────────┘
         │
         ▼                        S102
┌───────────────────────────────────────┐
│ CALCULATE FEATURE EXTRACTION PARAMETER │
│   ON BASIS OF FIRST FEATURE OBTAINED   │
│   THROUGH LEARNING AND STORE FEATURE   │
│ EXTRACTION PARAMETER IN PARAMETER STORAGE │
└───────────────────────────────────────┘
         │
         ▼
┌─────────────────┐
│       END       │
└─────────────────┘
```

# FIG. 4

```
┌─────────────────┐
│      START      │
└─────────────────┘
         │
         ▼                        S103
┌─────────────────────────────────┐
│       ACQUIRE FIRST IMAGE       │
└─────────────────────────────────┘
         │
         ▼                        S104
┌─────────────────────────────────┐
│     PREDICT POSITION OF MARKER  │
│        WITHIN FIRST IMAGE       │
└─────────────────────────────────┘
         │
         ▼                        S105
┌───────────────────────────────────────┐
│  ACQUIRE FEATURE EXTRACTION PARAMETER  │
│       AND CALCULATE LIKELIHOOD OF      │
│       PREDICTED POSITION OF MARKER ON  │
│  BASIS OF FIRST FEATURE REPRESENTED    │
│    BY FEATURE EXTRACTION PARAMETER     │
└───────────────────────────────────────┘
         │
         ▼                        S106
┌─────────────────────────────────┐
│    CALCULATE POSITION OF MARKER │
│        ON BASIS OF LIKELIHOOD   │
└─────────────────────────────────┘
         │
         ▼
┌─────────────────┐
│       END       │
└─────────────────┘
```

# FIG. 5

# FIG. 6

FIG. 7A

```
              ┌─────────────┐
              │    START    │
              └──────┬──────┘
                     │         ╭S210
              ┌──────▼──────────────┐
              │ ACQUIRE SECOND IMAGE │
              └──────┬──────────────┘
                     │         ╭S211
         ┌───────────▼─────────────────┐
         │   EXTRACT IMAGE INCLUDING    │
         │ MARKER IMAGE WITHIN SECOND IMAGE │
         └───────────┬─────────────────┘
                     │         ╭S212
      ┌──────────────▼──────────────────┐
      │ ACQUIRE TRAINING IMAGE ASSOCIATED │
      │   WITH EXTRACTED IMAGE INCLUDING  │
      │           MARKER IMAGE            │
      └──────────────┬──────────────────┘
                     │
              ┌──────▼──────┐
              │     END     │
              └─────────────┘
```

FIG. 7B

```
              ┌─────────────┐
              │    START    │
              └──────┬──────┘
                     │         ╭S220
              ┌──────▼──────────────┐
              │ ACQUIRE SECOND IMAGE │
              └──────┬──────────────┘
                     │         ╭S221
         ┌───────────▼─────────────────┐
         │  ESTIMATE POSTURE OF MARKER FROM │
         │ MARKER IMAGE WITHIN SECOND IMAGE │
         └───────────┬─────────────────┘
                     │         ╭S222
      ┌──────────────▼──────────────────┐
      │ ACQUIRE TRAINING IMAGE ASSOCIATED │
      │  WITH ESTIMATED POSTURE OF MARKER │
      └──────────────┬──────────────────┘
                     │
              ┌──────▼──────┐
              │     END     │
              └─────────────┘
```

# FIG. 8

# FIG. 9

FIG. 10

# FIG. 11

# FIG. 12A

```
                    ┌─────────────┐
                    │    START    │
                    └──────┬──────┘
                           │           ⟋S103
              ┌────────────▼────────────┐
              │   ACQUIRE FIRST IMAGE   │
              └────────────┬────────────┘
                           │           ⟋S104
              ┌────────────▼────────────┐
              │ PREDICT POSITION OF MARKER │
              │    WITHIN FIRST IMAGE    │
              └────────────┬────────────┘
                           │           ⟋S105
       ┌───────────────────▼───────────────────┐
       │ ACQUIRE FEATURE EXTRACTION PARAMETER   │
       │ AND CALCULATE LIKELIHOOD OF PREDICTED  │
       │    POSITION OF MARKER ON BASIS OF      │
       │      FIRST FEATURE REPRESENTED         │
       │  BY FEATURE EXTRACTION PARAMETER       │
       └───────────────────┬───────────────────┘
                           │           ⟋S106
              ┌────────────▼────────────┐
              │ CALCULATE POSITION OF MARKER ON │
              │    BASIS OF LIKELIHOOD   │
              └────────────┬────────────┘
                           │           ⟋S410
              ┌────────────▼────────────┐
              │  ESTIMATE POSTURE OF MARKER  │
              └────────────┬────────────┘
                           │           ⟋S411
              ┌────────────▼────────────┐
              │   SELECT TEMPLATE ASSOCIATED   │
              │ WITH ESTIMATED POSTURE OF MARKER │
              └────────────┬────────────┘
                           │
                    ┌──────▼──────┐
                    │     END     │
                    └─────────────┘
```

# FIG. 12B

```
                    ┌─────────────┐
                    │   START     │
                    └──────┬──────┘
                           │           ⌐S103
                    ┌──────▼──────────────┐
                    │ ACQUIRE FIRST IMAGE │
                    └──────┬──────────────┘
                           │           ⌐S104
                    ┌──────▼──────────────┐
                    │ PREDICT POSITION OF │
                    │ MARKER              │
                    │ WITHIN FIRST IMAGE  │
                    └──────┬──────────────┘
                           │           ⌐S420
                    ┌──────▼───────────────────┐
                    │ CALCULATE LIKELIHOOD OF   │
                    │ PREDICTED                 │
                    │ POSITION OF MARKER        │
                    │ USING SELECTED TEMPLATE   │
                    └──────┬───────────────────┘
                           │           ⌐S106
                    ┌──────▼──────────────┐
                    │ CALCULATE POSITION  │
                    │ OF MARKER           │
                    │ ON BASIS OF         │
                    │ LIKELIHOOD          │
                    └──────┬──────────────┘
                           │           ⌐S410
                    ┌──────▼──────────────┐
                    │ ESTIMATE POSTURE    │
                    │ OF MARKER           │
                    └──────┬──────────────┘
                           │           ⌐S411
                    ┌──────▼──────────────┐
                    │ SELECT TEMPLATE     │
                    │ ASSOCIATED          │
                    │ WITH ESTIMATED      │
                    │ POSTURE OF MARKER   │
                    └──────┬──────────────┘
                           │
                    ┌──────▼──────┐
                    │    END      │
                    └─────────────┘
```

# FIG. 13

A2

A1 (M)

# FIG. 14

500

110

**LEARNING DEVICE**

111
TRAINING IMAGE ACQUIRER

112
LEARNER

113
PARAMETER STORAGE

120

**MOVING OBJECT TRACKING DEVICE**

121
FIRST IMAGE ACQUIRER

122
TRACKER

540
CONTROLLER

SM

**EP 3 766 541 B1**

**Patent documents cited in the description**

- JP 2016131737 A
- WO 2018037608 A1
- US 2012238866 A1
- US 2012093397 A1